# EUROPEAN PATENT APPLICATION

(11) **EP 4 790 130 A1**
(43) Date of publication of application: **12.08.2026**
(21) Application number: 24874672.9
(22) Date of filing: 03.10.2024
(51) Int. Cl.: C12N 1/04, C08F 8/30, C08F 8/34, C08F 222/06

(54) **CRYOPROTECTANT FOR CELLS**

(30) Priority: 06.10.2023 JP 2023174728
(71) Applicant: NOF Corporation, Shibuya-ku Tokyo 150-6019 (JP)
(72) Inventor: YOSHIZAKI, Norihiro, Kawasaki-shi, Kanagawa 210-0821 (JP); KAWAHARA, Daiki, Kawasaki-shi, Kanagawa 210-0821 (JP); IMAMURA, Ryutaro, Kawasaki-shi, Kanagawa 210-0821 (JP); TAKAGI, Kengo, Kawasaki-shi, Kanagawa 210-0821 (JP); OHTAKE, Tomoyuki, Kawasaki-shi, Kanagawa 210-0821 (JP)
(74) Representative: J A Kemp LLP
(86) International application number: PCT/JP2024/035380
(87) International publication number: WO 2025/075072

(57) **Abstract**

The present invention provides a cryoprotectant for cells, containing an ampholytic polymer having a specific cationic group and a specific anionic group, a hydrophobic amino acid, and a water-soluble cellulose.

## Description

### [Technical Field]

The present invention relates to cryoprotectants that can reduce cell damage after cryopreservation and thawing of cells.

### [Background Art]

Cells constantly change due to time lapse and the surrounding environment. They need to be preserved properly to maintain their properties. At present, it is often achieved through cryopreservation. Conventionally, cell cryopreservation has been performed in a dispersed state. However, when cells are actually used, it is necessary to culture the cells in two or three dimensions to establish appropriate cell function and cell morphology. In recent years, many two-dimensional or three-dimensional culture methods, combining complicated structures or multiple cell types, have been developed to more closely resemble the in vivo environment, and the culture period tends to increase. Therefore, starting from cell culture each time cells are used requires time before the cells are actually used. Furthermore, as mentioned above, two-dimensional or three-dimensional culture methods, combining complicated structures or multiple cell types require skilled culture techniques, which hinders the widespread adoption of the culture techniques. Additionally, starting from cell culture each time cells are used, the results obtained include variations during the culture, which is undesirable from the perspective of obtaining uniform measurement results. Therefore, it is necessary to maintain not only cell viability after cryopreservation and thawing, but also cell function and cell morphology after cryopreservation and thawing.

Given the above circumstances, a cryoprotectant that can cryopreserve cells, cultured in two or three dimensions, while maintaining their morphology and function is desired. However, even when general cryoprotectants for dispersed cells, such as dimethyl sulfoxide (DMSO), are applied to cells cultured in two or three dimensions, the cells are almost entirely destroyed during after cryopreservation and thawing. Therefore, attempts are being made to develop new cryoprotectants that are applicable to cells cultured in two or three dimensions. In recent years, the development of cryoprotectants using ampholytic polymers has been actively performed.

For example, Patent Literature 1 reports that an ampholytic polymer represented by the following formula (8) has a cryoprotective effect on cells cultured in two dimensions, and that when this ampholytic polymer is used during cell cryopreservation, the cell viability after thawing is high.

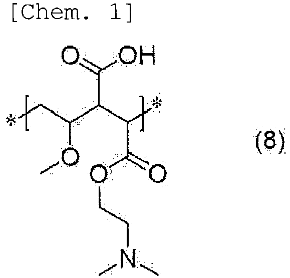

Furthermore, Non Patent Literature 1 reports that carboxylated polylysine (PLL-GA), represented by the following formula, has a cryoprotective effect on cells cultured in two or three dimensions, and that when the carboxylated polylysine is used during cell cryopreservation, the cell viability after thawing is high.

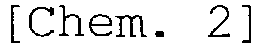

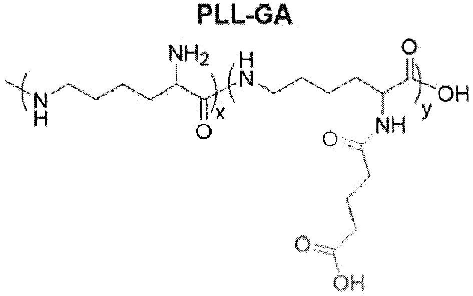

Ampholytic polymers are known to have an ice crystal formation inhibitory effect. By using ampholytic polymers during cell cryopreservation, ice crystal formation inside and outside the cell is inhibited, and the cell viability after thawing becomes high. However, there is a problem that using only ampholytic polymers makes it difficult to maintain the function and morphology of the cells after thawing.

In addition, Non Patent Literature 2 reports that proline has the effect of mitigating osmotic stress on cells during freezing, and that it shows high cell viability after thawing as a cryoprotective effect on cells cultured in two dimensions. However, there is a problem in that using only proline during cell cryopreservation makes it difficult to maintain the function and morphology of the cells after thawing.

### [Citation List]

### [Patent Literature]

[Patent Literature 1]
WO 2019/175596

### [Non Patent Literature]

[Non Patent Literature 1]
   Biomacromolecules 2020, 21, 3017-3025
[Non Patent Literature 2]
   Cryobiology 71 (2015) 472-480

### [Summary of Invention]

### [Technical Problem]

As described above, attempts have been made to develop cryoprotectants for cells cultured in two or three dimensions. While some have shown effectiveness in cell viability after thawing, the development of a cryoprotectant that can maintain the morphology and function of cells has not yet been achieved.

The problem of the present invention is to provide a cryoprotectant that can maintain not only cell viability but also morphology and function of cells (preferably cells cultured in two or three dimensions) after cryopreservation and thawing.

### [Solution to Problem]

The present inventors have conducted intensive studies in an attempt to achieve the aforementioned goal and found that the above-mentioned problem can be solved by using a cryoprotectant for cells that contains an ampholytic polymer having a specific cationic group and a specific anionic group, a hydrophobic amino acid, and a water-soluble cellulose (hereinafter sometimes abbreviated as "the cryoprotectant of the present invention"). Based on this finding, the present invention provides the following.
[1] A cryoprotectant for cells, comprising an ampholytic polymer, a hydrophobic amino acid, and a water-soluble cellulose, wherein
   the aforementioned ampholytic polymer has a cationic group selected from the group consisting of *-N⁺H₃, *-N⁺(CH₃)H₂, *-N⁺(CH₃)₂H, *-N⁺(CH₃)₃, *-N⁺(CH₃)₂-*, *-S⁺(CH₃)₂, and *-S⁺(CH₃)-* wherein, in the aforementioned formulas, * is a bonding position, and an anionic group selected from the group consisting of *-CO-O⁻, *-S(O)₂(O⁻), *-O-P(O)(OH)(O⁻), and *-OP(O)(O⁻)-O-* wherein, in the aforementioned formulas, * is a bonding position.
[2] The cryoprotectant of the aforementioned [1], wherein the aforementioned ampholytic polymer has a repeating structure A represented by the formula (1): 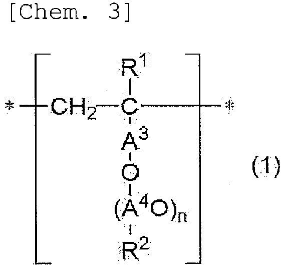
   wherein, in the formula (1), * is a bonding position,
      R¹ and R² are each independently a hydrogen atom or a methyl group,
      A³ is void or a methylene group,
      A⁴O is an oxyalkylene group having 2 to 4 carbon atoms, and
      n is a number between 0 and 100,
   a repeating structure B represented by the formula (2): 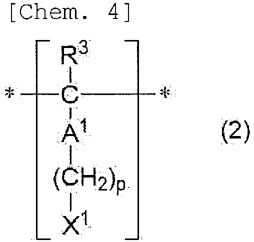
   wherein, in the formula (2), * is a bonding position,
      R³ is a hydrogen atom or a methyl group,
      A¹ is a group selected from the group consisting of *-COO-* and *-CO-NH-* wherein, in the aforementioned formulas, * is a bonding position,
      p is an integer of 1 to 6, and
      X¹ is a group selected from the group consisting of *-N⁺H₃, *-N⁺(CH₃)H₂, *-N⁺(CH₃)₂H, *-N⁺(CH₃)₃, and *-S⁺(CH₃)₂ wherein, in the aforementioned formulas, * is a bonding position, and a repeating structure C represented by the formula (3): 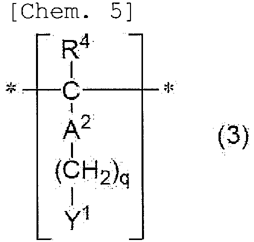
   wherein, in the formula (3), * is a bonding position,
      R⁴ is a hydrogen atom or a methyl group,
      A² is void or a group selected from the group consisting of *-CO-O-* and *-CO-NH-* wherein, in the aforementioned formulas, * is a bonding position,
      q is an integer of 0 to 6, and
      Y¹ is a group selected from the group consisting of *-COO⁻, *-S(O)₂(O⁻), *-O-P(O)(OH)(O⁻), and *-C₆H₄-S(O)₂(O⁻) wherein, in the aforementioned formulas, * is a bonding position and C₆H₄ is a phenylene group, and
      a molar ratio of the aforementioned repeating structure B to the total of the aforementioned repeating structure B and the aforementioned repeating structure C is 0.1 or more and 0.9 or less.
[3] The cryoprotectant of the aforementioned [2], wherein, in the aforementioned formula (1), R¹ is a hydrogen atom, R² is a methyl group, A³ is void or a methylene group, A⁴O is an oxyethylene group, and n is a number between 0 and 50,
   in the aforementioned formula (2), R³ is a hydrogen atom, A¹ is a group selected from the group consisting of *-CO-O-* and *-CO-NH-* wherein, in the aforementioned formulas, * is a bonding position, p is an integer of 2 to 4, and X¹ is a group selected from the group consisting of *-N⁺(CH₃)₂H and *-S⁺(CH₃)₂ wherein, in the aforementioned formulas, * is a bonding position, and
   in the aforementioned formula (3), R⁴ is a hydrogen atom, A² is void, q is 0, and Y¹ is *-CO-O⁻ wherein, in the aforementioned formula, * is a bonding position.
[4] The cryoprotectant of the aforementioned [3], wherein p is 2.
[5] The cryoprotectant of the aforementioned [1], wherein the aforementioned ampholytic polymer comprises a repeating structure D represented by the formula (4): 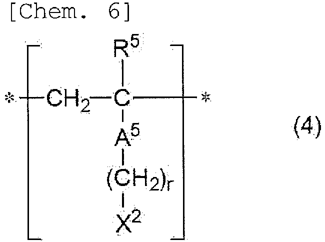
   wherein, in the formula (4), * is a bonding position,
      R⁵ is a hydrogen atom or a methyl group,
      A⁵ is a group selected from the group consisting of *-COO-* and *-CO-NH-* wherein, in the aforementioned formulas, * is a bonding position,
      r is an integer of 1 to 6, and
      X² is a group selected from the group consisting of *-N⁺H₃, *-N⁺(CH₃)H₂, *-N⁺(CH₃)₂H, *-N⁺(CH₃)₃, and *-S⁺(CH₃)₂ wherein, in the aforementioned formulas, * is a bonding position, and a repeating structure E represented by the formula (5): 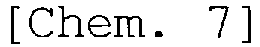 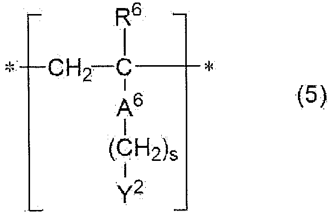
   wherein, in the formula (5), * is a bonding position,
      R⁶ is a hydrogen atom or a methyl group,
      A⁶ is void or a group selected from the group consisting of *-CO-O-*, and *-CO-NH-* wherein, in the aforementioned formulas, * is a bonding position,
      s is an integer of 0 to 6, and
      Y² is a group selected from the group consisting of *-COO⁻, *-S(O)₂(O⁻), *-O-P(O)(OH)(O⁻), and *-C₆H₄-S(O)₂(O⁻) wherein, in the aforementioned formulas, * is a bonding position and C₆H₄ is a phenylene group, and
      a molar ratio of the aforementioned repeating structure D to the total of the aforementioned repeating structure D and the aforementioned repeating structure E is 0.1 or more and 0.9 or less.
[6] The cryoprotectant of the aforementioned [5], wherein, in the aforementioned formula (4), R⁵ is a methyl group, A⁵ is *-CO-O-* wherein, in the aforementioned formula, * is a bonding position, r is 2, and X² is *-N⁺(CH₃)₂H wherein, in the aforementioned formula, * is a bonding position, and
   in the aforementioned formula (5), R⁶ is a methyl group, A⁶ is void or *-CO-O-* wherein, in the aforementioned formula, * is a bonding position, s is an integer of 0 to 3, and Y² is *-CO-O⁻ or *-S(O)₂(O⁻) wherein, in the aforementioned formulas, * is a bonding position.
[7] The cryoprotectant of the aforementioned [1], wherein the aforementioned ampholytic polymer has a group represented by the formula (6): 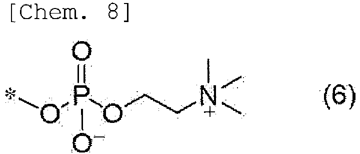 wherein, in the formula (6), * is a bonding position or a group represented by the formula (7): 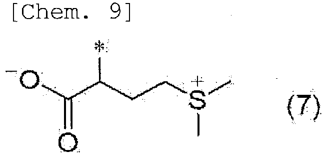 wherein, in the formula (7), * is a bonding position.
[8] The cryoprotectant of any one of the aforementioned [1] to [7], wherein the aforementioned ampholytic polymer has a number average molecular weight of 1,000 to 2,000,000.
[9] The cryoprotectant of any one of the aforementioned [1] to [8], wherein the aforementioned hydrophobic amino acid is proline.
[10] The cryoprotectant of any one of the aforementioned [1] to [9], wherein the aforementioned water-soluble cellulose is at least one selected from the group consisting of methylcellulose, hydroxyethylcellulose, hydroxypropylcellulose, and hydroxypropylmethylcellulose.
[11] A method for cryopreserving cells, comprising
   Step 1 of mixing cells with the cryoprotectant of any one of the aforementioned [1] to [10], and
   Step 2 of freezing the mixture containing the cells and the cryoprotectant.
[12] The method of the aforementioned [11], wherein the cells are cells cultured in two dimensions,
   the method comprises, before Step 1, a Step A of culturing adherent cells in two dimensions in a medium on a culture substrate,
   Step 1 is a Step C of mixing the cells cultured in two dimensions with the cryoprotectant of any one of the aforementioned [1] to [10], and
   Step 2 is a Step D of freezing a mixture containing the cells cultured in two dimensions on the culture substrate and the cryoprotectant.
[13] The method of the aforementioned [12], comprising, between Step A and Step C, a Step B of removing culture supernatant from the cells cultured in two dimensions.
[14] The method of the aforementioned [11], wherein the cells are cells cultured in three dimensions,
   the method comprises, before Step 1, a Step E of culturing adherent cells in three dimensions in a medium,
   Step 1 is a Step G of mixing the cells cultured in three dimensions with the cryoprotectant of any one of the aforementioned [1] to [10], and
   Step 2 is a Step H of freezing a mixture containing the cells cultured in three dimensions and the cryoprotectant.
[15] The method of the aforementioned [14], comprising, between Step E and Step G, a Step F of transferring the cells cultured in three dimensions to a cryopreservation container and removing culture supernatant.
[16] The method of the aforementioned [11], wherein cells suspended in a medium are cryopreserved,
   the method comprises, before Step 1, a Step I of suspending cells in a medium,
   Step 1 is a Step K, and
   Step 2 is a Step L.
[17] The method of the aforementioned [16], comprising, between Step I and Step K, a Step J of removing supernatant from the mixture containing the medium and the cells.

### [Advantageous Effects of Invention]

By using the cryoprotectant of the present invention, it is possible to maintain not only the cell viability but also the cell morphology and function after cryopreservation and thawing of cells.

### [Description of Embodiments]

The present invention is described in order below. The descriptions in the present specification can be combined with each other unless it is clear that they cannot be combined.

In the present specification, the "cryoprotectant for cells" means an additive that can reduce cell damage after cryopreservation and thawing of cells.

The cryoprotectant of the present invention contains an ampholytic polymer having a specific cationic group and a specific anionic group, a hydrophobic amino acid, and a water-soluble cellulose. Furthermore, the cryoprotectant of the present invention may contain water, medium, and other components to the extent that they do not inhibit the effects of the present invention. The cells and each component contained in the cryoprotectant of the present invention are described below.

### [Cell]

Examples of the cell include established cell for culture, and fertilized egg and egg cell from animals including human. Examples of the cell also include stem cells such as sperm cell, ES cell, iPS cell, mesenchymal stem cell, hematopoietic stem cell, neural stem cell, and umbilical cord blood cell, and animal cells including human cell, such as hepatocyte, nerve cell, cardiomyocyte, vascular endothelial cell, vascular smooth muscle cell, and blood cell. Examples of the cell also include plant cells.

In the present specification, that "cells are cultured in two dimensions" means culturing cells in a single layer on a culture substrate, and that "cells are cultured in three dimensions" means culturing cells with a thickness in the longitudinal direction.

In the present specification, "cells cultured in two dimensions" means cells obtained by two-dimensional culture, and "cells cultured in three dimensions" means cells obtained by three-dimensional culture.

The targets to be protected by the cryoprotectant of the present invention are preferably cells cultured in two dimensions, cells cultured in three dimensions, or cells suspended in a medium, more preferably cells cultured in two dimensions or cells cultured in three dimensions. Therefore, the cryoprotectant of the present invention is preferably a cryoprotectant for cells cultured in two dimensions, cells cultured in three dimensions, or cells suspended in a medium, more preferably for cells cultured in two dimensions or cells cultured in three dimensions.

Examples of the cell cultured in two dimensions include cells adhered to a culture substrate and monolayer cell membranes formed by adhesion of cells to each other. Examples of the cell cultured in three dimensions include spheroids and organoids formed by the aggregation and adhesion of cells, sheets of cells cultured in three dimensions formed by the stacking of the cells, and biological tissues. The constituent cells of cells cultured in two or three dimensions are not limited to one type, and they may be formed by mixing two or more types of cells.

From the viewpoint of cryoprotective effect, the amount of the cryoprotectant of the present invention is preferably 1 to 2,000 µL, more preferably 2 to 1,500 µL, further preferably 5 to 1,000 µL, per 1×10⁴ cells to be cryopreserved.

From the viewpoint of cryoprotective effect, the amount of the cryoprotectant of the present invention is preferably 10 to 300 µL, more preferably 20 to 200 µL, further preferably 50 to 200 µL, particularly preferably 50 to 100 µL, per 1×10⁴ cells cultured in two dimensions and to be cryopreserved.

From the viewpoint of cryoprotective effect, the amount of the cryoprotectant of the present invention is preferably 100 to 2,000 µL, more preferably 200 to 1,500 µL, further preferably 500 to 1,000 µL, per 1×10⁴ cells cultured in three dimensions and to be cryopreserved.

From the viewpoint of cryoprotective effect, the amount of the cryoprotectant of the present invention is preferably 1 to 20 µL, more preferably 2 to 15 µL, further preferably 5 to 10 µL, per 1×10⁴ cells suspended in a medium and to be cryopreserved.

### [Ampholytic polymer]

The ampholytic polymer used in the present invention may be either a polyampholite or a zwitterionic polymer. In the present specification, the "polyampholite" means a polymer in which cationic group and anionic group are not present in the same side chain (for example, a polymer having an anionic group as a pendant group and a cationic group in the side chain), and the "zwitterionic polymer" means a polymer in which cationic group and anionic group are present in the same side chain.

The ampholytic polymer used in the present invention has a cationic group selected from the group consisting of *-N⁺H₃, *-N⁺(CH₃)H₂, *-N⁺(CH₃)₂H, *-N⁺(CH₃)₃, *-N⁺(CH₃)₂-*, *-S⁺(CH₃)₂, and *-S⁺(CH₃)-* wherein, in the aforementioned formulas, * is a bonding position, and an anionic group selected from the group consisting of *-CO-O⁻, *-S(O)₂(O⁻), *-O-P(O)(OH)(O⁻), and *-OP(O)(O⁻)-O-* wherein, in the aforementioned formulas, * is a bonding position. As used herein, "*" in the aforementioned formulas or other formulas is a bonding position, not a carbon atom. Therefore, "*-" described in the present specification is a single bond.

In the present specification, groups represented by formulas using element symbols, parentheses, "-" and "*" have the same meaning as generally understood by those skilled in the art of chemistry. For example, "*-N⁺(CH₃)H₂" means a group in which one CH₃ and two H are bonded to N⁺ (i.e., methylammonio group), "*-S(O)₂(O⁻)" means a group in which two O and one O⁻ are bonded to S (i.e., sulfo group from which a proton has been removed), and "-P(O)(OH)(O⁻)" in "*-OP(O)(OH)(O⁻)" means a group in which O, OH and O⁻ are bonded to P (i.e., "*-O-P(O)(OH)(O⁻)" means a phosphate group from which one proton has been removed).

### <Polyampholite (ampholytic polymer 1)>

In one embodiment of the present invention, a preferred ampholytic polymer is a polyampholite having a repeating structure A represented by the formula (1): 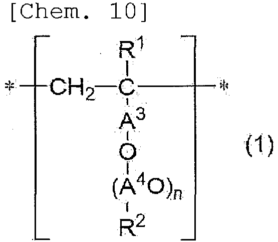
wherein, in the formula (1), * is a bonding position,
   R¹ and R² are each independently a hydrogen atom or a methyl group,
   A³ is void or a methylene group,
   A⁴O is an oxyalkylene group having 2 to 4 carbon atoms, and
   n is a number between 1 and 100,
a repeating structure B represented by the formula (2): 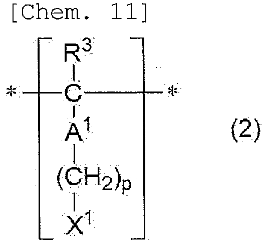
wherein, in the formula (2), * is a bonding position,
   R³ is a hydrogen atom or a methyl group,
   A¹ is a group selected from the group consisting of *-COO-* and *-CO-NH-* wherein, in the aforementioned formulas, * is a bonding position,
   p is an integer of 1 to 6, and
   X¹ is a group selected from the group consisting of *-N⁺H₃, *-N⁺(CH₃)H₂, *-N⁺(CH₃)₂H, *-N⁺(CH₃)₃, and *-S⁺(CH₃)₂ wherein, in the aforementioned formulas, * is a bonding position, and a repeating structure C represented by the formula (3): 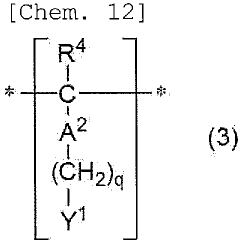
wherein, in the formula (3), * is a bonding position,
   R⁴ is a hydrogen atom or a methyl group,
   A² is void or a group selected from the group consisting of *-CO-O-* and *-CO-NH-* wherein, in the aforementioned formulas, * is a bonding position,
   q is an integer of 0 to 6, and
   Y¹ is a group selected from the group consisting of *-COO⁻, *-S(O)₂(O⁻), *-O-P(O)(OH)(O⁻), and *-C₆H₄-S(O)₂(O⁻) wherein, in the aforementioned formulas, * is a bonding position and C₆H₄ is a phenylene group (sometimes referred to as "ampholytic polymer 1" in the present specification).

*-CO-O-* and *-CO-NH-*, which are A¹ in the formula (2), are both bonded to the carbon atom (C) above A¹ in the formula (2) on the "*-CO" side. From the viewpoint of stability of ampholytic polymer 1, A¹ in the formula (2) is preferably *-CO-NH-*.

*-CO-O-* and *-CO-NH-*, which are A² in the formula (3), are both bonded to the carbon atom (C) above A² in the formula (3) on the "*-CO" side.

In the present specification, "repeating structure" means a substructure that appears repeatedly in the polymer. This repeating structure may be a repeating unit itself or a part of a repeating unit.

In the present specification, "repeating unit" means a substructure formed from a monomer that appears repeatedly in the polymer. Therefore, when multiple types of monomers polymerize to form a polymer, the ratio of each monomer to the total monomers is equal to the ratio of the repeating unit formed from the aforementioned monomer to the total repeating units.

In the present specification, "A³ is void" means that the carbon atom (C) above A³ in the formula (1) and the oxygen atom (O) therebelow are bonded by a single bond. Other expressions similar to "A³ is void" have the same meaning.

In the present specification, "n is 0" means that (A⁴O)ₙ in the formula (1) is void, and the oxygen atom (O) above (A⁴O)ₙ in the formula (1) and R² therebelow are bonded by a single bond. Other expressions similar to "n is 0" have the same meaning. Also, n in the formula (1) is the number of added oxyalkylene groups (A⁴O) having 2 to 4 carbon atoms, and is practically an average value. Therefore, n may be a decimal.

Examples of repeating structure A represented by the aforementioned formula (1) include the following:
repeating structure A wherein R¹ is a hydrogen atom, R² is a methyl group, A³ is void, and n is 0, and
repeating structure A wherein R¹ is a hydrogen atom, R² is a methyl group, A³ is a methylene group, A⁴O is an oxyalkylene group having 2 to 4 carbon atoms (preferably 2 carbon atoms), and n is a number between 5 and 50 (preferably between 5 and 45).

From the viewpoint of solubility of ampholytic polymer 1 in water, for example, preferred repeating structure A includes the following:
repeating structure A wherein R¹ is a hydrogen atom, R² is a methyl group, A³ is void, and n is 0, and
repeating structure A wherein R¹ is a hydrogen atom, R² is a methyl group, A³ is a methylene group, A⁴O is an oxyalkylene group having 2 or 3 carbon atoms (preferably 2 carbon atoms), and n is a number between 5 and 50 (preferably between 5 and 45).

From the viewpoint of solubility of the ampholytic polymer in water, for example, more preferred repeating structure A includes the following:
repeating structure A wherein R¹ is a hydrogen atom, R² is a methyl group, A³ is void, and n is 0, and
repeating structure A wherein R¹ is a hydrogen atom, R² is a methyl group, A³ is a methylene group, A⁴O is an oxyethylene group, and n is a number between 5 and 50 (preferably between 5 and 45).

Examples of repeating structure B represented by the aforementioned formula (2) include the following (in the following formulas, * is a bonding position):
repeating structure B wherein R³ is a hydrogen atom, A¹ is *-CO-O-*, p is an integer of 2 to 4 (preferably p is 2), and X¹ is *-N⁺(CH₃)₂H,
repeating structure B wherein R³ is a hydrogen atom, A¹ is *-CO-NH-*, p is an integer of 2 to 4 (preferably p is 2), and X¹ is *-N⁺(CH₃)₂H,
repeating structure B wherein R³ is a hydrogen atom, A¹ is *-CO-O-*, p is an integer of 2 to 4 (preferably p is 2), and X¹ is *-S⁺(CH₃)₂,
repeating structure B wherein R³ is a hydrogen atom, A¹ is *-CO-NH-*, p is an integer of 2 to 4 (preferably p is 2), and X¹ is *-S⁺(CH₃)₂,
repeating structure B wherein R³ is a hydrogen atom, A¹ is *-CO-O-*, p is an integer of 2 to 4 (preferably p is 2), and X is *-N⁺H₃, and
repeating structure B wherein R³ is a hydrogen atom, A¹ is *-CO-NH-*, p is an integer of 2 to 4 (preferably p is 2), and X is *-N⁺H₃.

From the viewpoint of solubility of ampholytic polymer 1 in water, for example, preferred repeating structure B includes the following (in the following formulas, * is a bonding position):
repeating structure B wherein R³ is a hydrogen atom, A¹ is *-CO-O-*, p is an integer of 2 to 4 (preferably p is 2), and X¹ is *-N⁺(CH₃)₂H,
repeating structure B wherein R³ is a hydrogen atom, A¹ is *-CO-NH-*, p is an integer of 2 to 4 (preferably p is 2), and X¹ is *-N⁺(CH₃)₂H,
repeating structure B wherein R³ is a hydrogen atom, A¹ is *-CO-O-*, p is an integer of 2 to 4 (preferably p is 2), and X¹ is *-S⁺(CH₃)₂, and
repeating structure B wherein R³ is a hydrogen atom, A¹ is *-CO-NH-*, p is an integer of 2 to 4 (preferably p is 2), and X¹ is *-S⁺(CH₃)₂.

From the viewpoint of solubility of ampholytic polymer 1 in water, for example, more preferred repeating structure B includes the following (in the following formulas, * is a bonding position):
repeating structure B wherein R³ is a hydrogen atom, A¹ is *-CO-NH-*, p is an integer of 2 to 4 (preferably p is 2), and X¹ is *-N⁺(CH₃)₂H, and
repeating structure B wherein R³ is a hydrogen atom, A¹ is *-CO-O-*, p is an integer of 2 to 4 (preferably p is 2), and X¹ is *-S⁺(CH₃)₂.

Examples of repeating structure C represented by the aforementioned formula (3) include the following (in the following formulas, * is a bonding position):
repeating structure C wherein R⁴ is a hydrogen atom, A² is void, q is 0, and Y¹ is *-CO-O⁻,
repeating structure C wherein R⁴ is a hydrogen atom, A² is *-CO-O-*, q is 2, and Y¹ is *-CO-O⁻,
repeating structure C wherein R⁴ is a hydrogen atom, A² is *-CO-O-*, q is 2, and Y¹ is *-S(O)₂(O⁻),
repeating structure C wherein R⁴ is a hydrogen atom, A² is *-CO-O-*, q is 2, and Y¹ is *-O-P(O)(OH)(O⁻), and
repeating structure C wherein R⁴ is a hydrogen atom, A² is void, q is 0, and Y¹ is *-C₆H₄-S(O)₂(O⁻).

From the viewpoint of easy synthesis of ampholytic polymer 1, for example, preferred repeating structure C includes the following (in the following formulas, * is a bonding position, and C₆H₄ is a phenylene group):
repeating structure C wherein R⁴ is a hydrogen atom, A² is void, q is 0, and Yⁱ is *-CO-O⁻,
repeating structure C wherein R⁴ is a hydrogen atom, A² is *-CO-O-*, q is 2, and Y¹ is *-CO-O⁻, and
repeating structure C wherein R⁴ is a hydrogen atom, A² is void, q is 0, and Y¹ is *-C₆H₄-S(O)₂(O⁻).

From the viewpoint of easy synthesis of ampholytic polymer 1, for example, more preferred repeating structure C includes the following (in the following formulas, * is a bonding position):
repeating structure C wherein R⁴ is a hydrogen atom, A² is void, q is 0, and Y¹ is *-CO-O⁻, and
repeating structure C wherein R⁴ is a hydrogen atom, A² is *-CO-O-*, q is 2, and Y¹ is *-CO-O⁻.

From the viewpoint of easy synthesis of ampholytic polymer 1, for example, further preferred repeating structure C includes the following (in the following formula, * is a bonding position):
repeating structure C wherein R⁴ is a hydrogen atom, A² is void, q is 0, and Y¹ is *-CO-O⁻.

From the viewpoint of cell cryoprotective effect of ampholytic polymer 1, it is preferable that
in the aforementioned formula (1), R¹ is a hydrogen atom, R² is a methyl group, A³ is void or a methylene group, A⁴O is an oxyethylene group, and n is a number between 0 and 50 (more preferably 5 and 50, further preferably 5 and 45),
in the aforementioned formula (2), R³ is a hydrogen atom, A¹ is a group selected from the group consisting of *-CO-O-* and *-CO-NH-* wherein, in the aforementioned formulas, * is a bonding position (more preferably *-CO-NH-*), p is an integer of 2 to 4 (preferably p is 2), and X¹ is a group selected from the group consisting of *-N⁺(CH₃)₂H and *-S⁺(CH₃)₂ wherein, in the aforementioned formulas, * is a bonding position, and
in the aforementioned formula (3), R⁴ is a hydrogen atom, A² is void, q is 0, and Y¹ is *-CO-O⁻ wherein, in the aforementioned formula, * is a bonding position.

From the viewpoint of cryoprotective effect and cytotoxicity of ampholytic polymer 1, the molar ratio of the aforementioned repeating structure B to the total of the aforementioned repeating structure B and the aforementioned repeating structure C (i.e., amount of the aforementioned repeating structure B (mol)/(amount of the aforementioned repeating structure B (mol) + amount of the aforementioned repeating structure C (mol))) is preferably 0.1 or more and 0.9 or less. When the aforementioned molar ratio is less than 0.1, the ampholytic polymer 1 becomes strongly anionic, may not remain around cells, and the cryoprotective effect thereof may not be exerted. When the aforementioned molar ratio exceeds 0.9, the ampholytic polymer 1 becomes strongly cationic and may show cytotoxicity. From the viewpoints of cryoprotective effect and cytotoxicity of ampholytic polymer 1, the aforementioned molar ratio is more preferably 0.2 or more and 0.7 or less, further preferably 0.2 or more and 0.5 or less.

From the viewpoint of cryoprotective effect of ampholytic polymer 1, the proportion of repeating structure A in ampholytic polymer 1 is preferably 70 mol% or less, more preferably 60 mol% or less, relative to the total repeating units of ampholytic polymer 1. The repeating structure A is generally a repeating unit.

From the viewpoint of solubility of ampholytic polymer 1 in water, the proportion of repeating structure A in ampholytic polymer 1 is preferably 25 mol% or more, more preferably 35 mol% or more, relative to the total repeating units of ampholytic polymer 1.

In ampholytic polymer 1, repeating structure B is preferably bonded to repeating structure C. That is, ampholytic polymer 1 preferably contains repeating structure B-C represented by the formula (2-3): 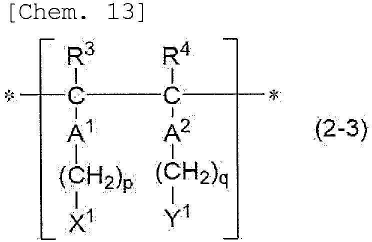 wherein the symbols in the formula (2-3) are as defined in the formula (2) and the formula (3). The repeating structure B-C is generally a repeating unit.

In ampholytic polymer 1, two repeating structures C may be bonded. That is, ampholytic polymer 1 may contain repeating structure C-C represented by the formula (3-3): 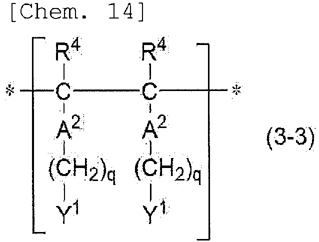 wherein the symbols in the formula (3-3) are as defined in the formula (3). The repeating structure C-C is generally a repeating unit.

The ampholytic polymer 1 may contain structures different from the aforementioned repeating structures A to C. For example, ampholytic polymer 1 may contain a repeating unit different from any of the aforementioned repeating structure A (= repeating unit), a repeating unit including the aforementioned repeating structure B, and a repeating unit including the aforementioned repeating structure C (hereinafter sometimes referred to as "other repeating unit of ampholytic polymer 1").

Examples of monomers for forming other repeating unit of ampholytic polymer 1 (hereinafter sometimes referred to as "other monomer for ampholytic polymer 1") include the following:
(meth)acrylates such as diethylaminoethyl (meth)acrylate, butyl (meth)acrylate, polyethylene glycol mono(meth)acrylate, glycerol (meth)acrylate, (meth)acryloyloxyethylphosphate, (meth)acryloyloxyethyl phosphorylcholine, N-methylcarboxybetaine (meth)acrylate, N-methylsulfobetaine (meth)acrylate, methyl (meth)acrylate, glycidyl (meth)acrylate, dimethylaminoethyl (meth)acrylate, 2-hydroxyethyl methacrylate, 2-methoxyethyl (meth)acrylate, and the like; and
radical polymerizable monomers such as acrylamide, N,N'-dimethylacrylamide, N-isopropyl (meth)acrylamide, (meth)acrylic acid, allyl alcohol, acrylonitrile, acrolein, vinyl acetate, sodium vinylsulfonate, styrene, chlorostyrene, vinylphenol, vinyl cinnamate, vinyl chloride, vinyl bromide, butadiene, vinylene carbonate, itaconic acid, itaconate, fumaric acid, fumarate, maleic acid, maleate, maleic anhydride, and the like.

In the present specification, "(meth)acrylic acid" basically means acrylic acid or methacrylic acid. When multiple "(meth)acrylic acids" may exist, "(meth)acrylic acid" means acrylic acid and/or methacrylic acid. Other terms similar to "(meth)acrylic acid" have the same meaning.

As other monomers for ampholytic polymer 1, from the viewpoint of solubility of ampholytic polymer 1, styrene, maleic anhydride, N,N'-dimethylacrylamide, butyl (meth)acrylate, and polyethylene glycol mono(meth)acrylate are preferred, and styrene and maleic anhydride are more preferred.

To sufficiently bring out the property of ampholytic polymer 1, the proportion of other repeating units in ampholytic polymer 1 is preferably 30 mol% or less, more preferably 10 mol% or less, further preferably 5 mol% or less, relative to the total repeating units of ampholytic polymer 1. The ampholytic polymer 1 does not necessarily contain other repeating units.

When other repeating units (e.g., repeating unit formed from styrene, repeating unit formed from maleic anhydride) of ampholytic polymer 1 are included in ampholytic polymer 1, from the viewpoint of the solubility of ampholytic polymer 1 in water, the proportion of other repeating units in the ampholytic polymer 1 is preferably 0.1 mol% or more, more preferably 0.5 mol% or more, relative to the total repeating units of ampholytic polymer 1.

The ampholytic polymer 1 may be an alternating copolymer, a random copolymer, or a block copolymer. For example, it may be a copolymer having the structure of an alternating copolymer and the structure of a random copolymer. From the viewpoint of easy synthesis, ampholytic polymer 1 is preferably an alternating copolymer or a random copolymer, more preferably a random copolymer.

### <Polyampholite (ampholytic polymer 2)>

In one embodiment of the present invention, a preferred ampholytic polymer is a polyampholite having a repeating structure D represented by the formula (4): 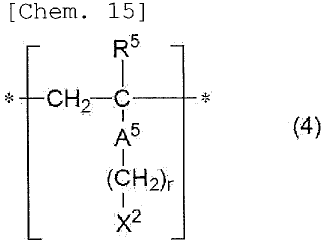
wherein, in the formula (4), * is a bonding position,
   R⁵ is a hydrogen atom or a methyl group,
   A⁵ is a group selected from the group consisting of *-COO-* and *-CO-NH-* wherein, in the aforementioned formulas, * is a bonding position,
   r is an integer of 1 to 6, and
   X² is a group selected from the group consisting of *-N⁺H₃, *-N⁺(CH₃)H₂, *-N⁺(CH₃)₂H, *-N⁺(CH₃)₃, and *-S⁺(CH₃)₂ wherein, in the aforementioned formulas, * is a bonding position, and a repeating structure E represented by the formula (5): 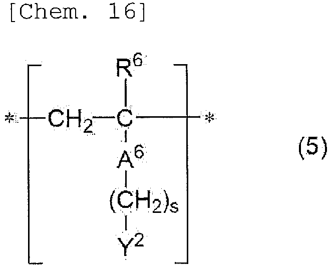
   wherein, in the formula (5), * is a bonding position,
      R⁶ is a hydrogen atom or a methyl group,
      A⁶ is void or a group selected from the group consisting of *-CO-O-* and *-CO-NH-* wherein, in the aforementioned formulas, * is a bonding position,
      s is an integer of 0 to 6, and
      Y² is a group selected from the group consisting of *-COO⁻, -S(O)₂(O⁻), *-O-P(O)(OH)(O⁻), and *-C₆H₄-S(O)₂(O⁻) wherein, in the aforementioned formulas, * is a bonding position and C₆H₄ is a phenylene group)
   (sometimes referred to as "ampholytic polymer 2" in the present specification).

*-CO-O-* and *-CO-NH-*, which are A⁵ in the formula (4), are both bonded to the carbon atom (C) above A⁵ in the formula (4) on the "*-CO" side.

*-CO-O-* and *-CO-NH-*, which are A⁶ in the formula (5), are both bonded to the carbon atom (C) above A⁶ in the formula (4) on the "*-CO" side.

Examples of repeating structure D represented by the aforementioned formula (4) include the following (in the following formulas, * is a bonding position):
repeating structure D wherein R⁵ is a methyl group, A⁵ is *-CO-O-*, r is 2, and X² is *-N⁺(CH₃)₂H,
repeating structure D wherein R⁵ is a methyl group, A⁵ is *-CO-O-*, r is 2, and X² is *-N⁺H₃,
repeating structure D wherein R⁵ is a methyl group, A⁵ is *-CO-O-*, r is 2, and X² is *-N⁺(CH₃)₃,
repeating structure D wherein R⁵ is a methyl group, A⁵ is *-CO-O-*, r is 2, and X² is *-S⁺(CH₃)₂,
repeating structure D wherein R³ is a hydrogen atom, A⁵ is *-CO-O-*, r is 2, and X² is *-N⁺(CH₃)₂H,
repeating structure D wherein R⁵ is a hydrogen atom, A⁵ is *-CO-O-*, r is 2, and X² is *-N⁺H₃,
repeating structure D wherein R⁵ is a hydrogen atom, A⁵ is *-CO-O-*, r is 2, and X² is *-N⁺(CH₃)₃,
repeating structure D wherein R⁵ is a hydrogen atom, A⁵ is *-CO-O-*, r is 2, and X² is *-S⁺(CH₃)₂,
repeating structure D wherein R⁵ is a hydrogen atom, A⁵ is *-CO-NH-*, r is 2, X² is *-N⁺(CH₃)₂H,
repeating structure D wherein R⁵ is a hydrogen atom, A⁵ is *-CO-NH-*, r is 2, and X² is *-N⁺H₃, and
repeating structure D wherein R⁵ is a hydrogen atom, A⁵ is *-CO-NH-*, r is 2, and X² is *-S⁺(CH₃)₂.

From the viewpoint of easy synthesis of ampholytic polymer 2, for example, preferred repeating structure D includes the following (in the following formulas, * is a bonding position):
repeating structure D wherein R⁵ is a methyl group, A⁵ is *-CO-O-*, r is 2, and X² is *-N⁺(CH₃)₂H,
repeating structure D wherein R⁵ is a methyl group, A⁵ is *-CO-O-*, r is 2, and X² is *-N⁺H₃,
repeating structure D wherein R⁵ is a methyl group, A⁵ is *-CO-O-*, r is 2, and X² is *-N⁺(CH₃)₃,
repeating structure D wherein R⁵ is a methyl group, A⁵ is *-CO-O-*, r is 2, and X² is *-S⁺(CH₃)₂,
repeating structure D wherein R⁵ is a hydrogen atom, A⁵ is *-CO-O-*, r is 2, and X² is *-N⁺(CH₃)₂H,
repeating structure D wherein R⁵ is a hydrogen atom, A⁵ is *-CO-O-*, r is 2, and X² is *-N⁺H₃,
repeating structure D wherein R⁵ is a hydrogen atom, A⁵ is *-CO-NH-*, r is 2, and X² is *-N⁺H₃, and
repeating structure D wherein R⁵ is a hydrogen atom, A⁵ is *-CO-NH-*, r is 2, and X² is *-S⁺(CH₃)₂.

From the viewpoint of easy synthesis of ampholytic polymer 2, for example, more preferred repeating structure D includes the following (in the following formulas, * is a bonding position):
repeating structure D wherein R⁵ is a methyl group, A⁵ is *-CO-O-*, r is 2, and X² is *-N⁺(CH₃)₂H,
repeating structure D wherein R⁵ is a methyl group, A⁵ is *-CO-O-*, r is 2, and X² is *-N⁺H₃,
repeating structure D wherein R⁵ is a methyl group, A⁵ is *-CO-O-*, r is 2, and X² is *-N⁺(CH₃)₃, and
repeating structure D wherein R⁵ is a methyl group, A⁵ is *-CO-O-*, r is 2, and X² is *-S⁺(CH₃)₂.

From the viewpoint of easy synthesis of ampholytic polymer 2, for example, further preferred repeating structure D includes the following (in the following formulas, * is a bonding position):
repeating structure D wherein R⁵ is a methyl group, A⁵ is *-CO-O-*, r is 2, and X² is *-N⁺(CH₃)₂H.

Examples of repeating structure E represented by the aforementioned formula (5) include the following (in the following formulas, * is a bonding position and C₆H₄ is a phenylene group):
repeating structure E wherein R⁶ is a methyl group, A⁶ is void, s is an integer of 0 to 3 (preferably 0), and Y² is *-COO⁻,
repeating structure E wherein R⁶ is a hydrogen atom, A⁶ is void, s is an integer of 0 to 3 (preferably 0), and Y² is *-C₆H₄-S(O)₂(O⁻),
repeating structure E wherein R⁶ is a methyl group, A⁶ is *-CO-O-*, s is an integer of 0 to 3 (preferably 2), and Y² is *-CO-O⁻,
repeating structure E wherein R⁶ is a methyl group, A⁶ is *-CO-O-*, s is an integer of 0 to 3 (preferably 2), and Y² is *-S(O)₂(O⁻),
repeating structure E wherein R⁶ is a methyl group, A⁶ is *-CO-O-*, s is an integer of 0 to 3 (preferably 2), and Y² is *-O-P(O)(OH)(O⁻), and
repeating structure E wherein R⁶ is a hydrogen atom, A⁶ is void, s is an integer of 0 to 3 (preferably 0), and Y² is *-CO-O⁻.

From the viewpoint of easy synthesis of ampholytic polymer 2, for example, preferred repeating structure E includes the following (in the following formulas, * is a bonding position and C₆H₄ is a phenylene group):
repeating structure E wherein R⁶ is a methyl group, A⁶ is void, s is 0, and Y² is *-CO-O⁻,
repeating structure E wherein R⁶ is a hydrogen atom, A⁶ is void, s is 0, and Y² is *-C₆H₄-S(O)₂(O⁻),
repeating structure E wherein R⁶ is a hydrogen atom, A⁶ is void, s is 0, and Y² is *-CO-O⁻, and
repeating structure E wherein R⁶ is a methyl group, A⁶ is *-CO-O-*, s is 2 or 3 (preferably 3), and Y² is *-CO-O⁻.

From the viewpoint of easy synthesis of ampholytic polymer 2, for example, more preferred repeating structure E includes the following (in the following formulas, * is a bonding position):
repeating structure E wherein R⁶ is a methyl group, A⁶ is void, s is 0, and Y² is *-CO-O⁻,
repeating structure E wherein R⁶ is a hydrogen atom, A⁶ is void, s is 0, and Y² is *-CO-O⁻, and
repeating structure E wherein R⁶ is a methyl group, A⁶ is *-CO-O-*, s is 2 or 3 (preferably 3), and Y² is *-CO-O⁻.

From the viewpoint of easy synthesis of ampholytic polymer 2, for example, further preferred repeating structure E includes the following (in the following formulas, * is a bonding position):
repeating structure E wherein R⁶ is a methyl group, A⁶ is void, s is 0, and Y² is *-CO-O⁻, and
repeating structure E wherein R⁶ is a methyl group, A⁶ is *-CO-O-*, s is 3, and Y² is *-CO-O⁻.

From the viewpoint of easy synthesis of ampholytic polymer 2, it is preferable that
in the aforementioned formula (4), R⁵ is a methyl group, A⁵ is *-CO-O-* wherein, in the aforementioned formula, * is a bond ing position, r is 2, and X² is *-N⁺(CH₃)₂H wherein,
   in the aforementioned formula, * is a bonding position, and
in the aforementioned formula (5), R⁶ is a methyl group, A⁶ is void or *-CO-O-* wherein, in the aforementioned formula, * is a bonding position, s is an integer of 0 to 3 (more preferably 0), and Y² is *-CO-O⁻ or *-S(O)₂(O⁻) wherein, in the aforementioned formula, * is a bonding position.

From the viewpoint of easy synthesis of ampholytic polymer 2, it is more preferable that
in the aforementioned formula (4), R⁵ is a methyl group, A⁵ is *-CO-O-* wherein, in the aforementioned formula, * is a bonding position, r is 2, and X² is *-N⁺(CH₃)₂H wherein, in the aforementioned formula, * is a bonding position, and
in the aforementioned formula (5), R⁶ is a methyl group, A⁶ is void, s is 0, and Y² is *-CO-O⁻ wherein, in the aforementioned formula, * is a bonding position.

From the viewpoint of the achievement of cryoprotective effect and cytotoxicity of ampholytic polymer 2, the molar ratio of the aforementioned repeating structure D to the total of the aforementioned repeating structure D and the aforementioned repeating structure E (i.e., amount of the aforementioned repeating structure D (mol)/(amount of the aforementioned repeating structure D (mol) + amount of the aforementioned repeating structure E (mol))) is preferably 0.1 or more and 0.9 or less. When the aforementioned molar ratio is less than 0.1, the ampholytic polymer 2 becomes strongly anionic, may not remain around cells, and the cryoprotective effect thereof may not be exerted. When the aforementioned molar ratio exceeds 0.9, the ampholytic polymer 2 becomes strongly cationic and may show cytotoxicity. From the viewpoints of cryoprotective effect and cytotoxicity of ampholytic polymer 2, the aforementioned molar ratio is more preferably 0.2 or more and 0.7 or less, further preferably 0.2 or more and 0.6 or less, particularly preferably 0.2 or more and 0.5 or less.

The ampholytic polymer 2 may contain structures different from the aforementioned repeating structures D and E. For example, ampholytic polymer 2 may contain a repeating unit different from any of the aforementioned repeating structures D and E (hereinafter sometimes referred to as "other repeating unit of ampholytic polymer 2"). Both the aforementioned repeating structures D and E are generally repeating units.

Examples of monomers for forming other repeating unit of ampholytic polymer 2 (hereinafter sometimes referred to as "other monomer for ampholytic polymer 2") include the following:
(meth)acrylates such as butyl (meth)acrylate, polyethylene glycol mono(meth)acrylate, glycerol (meth)acrylate, (meth)acryloyloxyethylphosphate, (meth)acryloyloxyethyl phosphorylcholine, N-methylcarboxybetaine (meth)acrylate, N-methylsulfobetaine (meth)acrylate, methyl (meth)acrylate, glycidyl (meth)acrylate, 2-hydroxyethyl methacrylate, 2-methoxyethyl (meth)acrylate, and the like;
vinyl ethers such as methyl vinyl ether and the like; and
various radical polymerizable monomers such as acrylamide, N,N'-dimethylacrylamide, N-isopropyl (meth)acrylamide, allyl alcohol, acrylonitrile, acrolein, vinyl acetate, sodium vinylsulfonate, styrene, chlorostyrene, vinylphenol, vinyl cinnamate, vinyl chloride, vinyl bromide, butadiene, vinylene carbonate, itaconic acid, itaconate, fumaric acid, fumarate, maleic acid, maleate, and the like.

As other monomers for ampholytic polymer 2, from the viewpoint of the solubility of ampholytic polymer 2, N,N'-dimethylacrylamide, butyl (meth)acrylate, and polyethylene glycol mono(meth)acrylate are preferred, and butyl (meth)acrylate is more preferred.

To sufficiently bring out the property of ampholytic polymer 2, the proportion of other repeating units in ampholytic polymer 2 is preferably 30 mol% or less, more preferably 10 mol% or less, relative to the total repeating units of ampholytic polymer 2. The ampholytic polymer 2 does not necessarily contain other repeating units.

When other repeating units (for example, repeating units formed from butyl (meth)acrylate) of ampholytic polymer 2 are included in ampholytic polymer 2, from the viewpoint of the cryoprotective effect of ampholytic polymer 2, the proportion of other repeating units in ampholytic polymer 2 is preferably 1 mol% or more, more preferably 5 mol% or more, relative to the total repeating units of ampholytic polymer 2.

The ampholytic polymer 2 may be an alternating copolymer, a random copolymer, or a block copolymer. For example, it may be a copolymer having the structure of an alternating copolymer and the structure of a random copolymer. From the viewpoint of easy synthesis, ampholytic polymer 2 is preferably a random copolymer.

### <Zwitterionic polymer>

Examples of the combination of the cationic group and anionic group in the zwitterionic polymer include the following (in the following formulas, * is a bonding position):
combination of *-N⁺(CH₃)₃ and *-O-P(O)(O⁻)-O-*,
combination of *-N⁺(CH₃)₂-* and *-CO-O⁻,
combination of *-N⁺(CH₃)₂-* and *-S(O)₂(O⁻),
combination of *-N⁺(CH₃)₂-* and *-O-P(O)(OH)(O⁻),
combination of *-S⁺(CH₃)₂ and *-CO-O⁻, and
combination of *-S⁺(CH₃)₂ and *-S(O)₂(O⁻).

From the viewpoint of easy synthesis of zwitterionic polymers, for example, preferred combination of the cationic group and anionic group in the zwitterionic polymer includes the following (in the following formulas, * is a bonding position):
combination of *-N⁺(CH₃)₃ and *-O-P(O)(O⁻)-O-*,
combination of *-N⁺(CH₃)₂-* and *-CO-O⁻,
combination of *-N⁺(CH₃)₂-* and *-S(O)₂(O⁻), and
combination of *-S⁺(CH₃)₂ and *-CO-O⁻.

From the viewpoint of easy synthesis of zwitterionic polymers, for example, more preferred combination of the cationic group and anionic group in the zwitterionic polymer includes the following (in the following formulas, * is a bonding position):
combination of *-N⁺(CH₃)₃ and *-O-P(O)(O⁻)-O-*, and
combination of *-S⁺(CH₃)₂ and *-CO-O⁻.

### <Zwitterionic polymer (ampholytic polymer 3)>

In one embodiment of the present invention, a preferred ampholytic polymer is a zwitterionic polymer having a group represented by the formula (6): 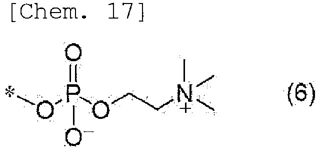
wherein, in the formula (6), * is a bonding position, or a group represented by the formula (7): 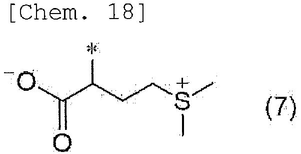
wherein, in the formula (7), * is a bonding position (sometimes referred to as "ampholytic polymer 3" in the present specification).

The ampholytic polymer 3 may contain a repeating unit different from any of a repeating unit having a group represented by the aforementioned formula (6) and a repeating unit having a group represented by the aforementioned formula (7) (hereinafter sometimes referred to as "other repeating unit of ampholytic polymer 3").

Examples of monomers for forming other repeating unit of ampholytic polymer 3 (hereinafter sometimes referred to as "other monomer for ampholytic polymer 3") include the following:
(meth)acrylates such as diethylaminoethyl (meth)acrylate, butyl (meth)acrylate, polyethylene glycol mono(meth)acrylate, glycerol (meth)acrylate, (meth)acryloyloxyethylphosphate methyl (meth)acrylate, glycidyl (meth)acrylate, dimethylaminoethyl (meth)acrylate, 2-hydroxyethyl methacrylate, 2-methoxyethyl (meth)acrylatem, and the like;
vinyl ethers such as methyl vinyl ether and the like; and
various radical polymerizable monomers such as acrylamide, N,N'-dimethylacrylamide, N-isopropyl (meth)acrylamide, (meth)acrylic acid, allyl alcohol, acrylonitrile, acrolein, vinyl acetate, sodium vinylsulfonate, styrene, chlorostyrene, vinylphenol, vinyl cinnamate, vinyl chloride, vinyl bromide, butadiene, vinylene carbonate, itaconic acid, itaconate, fumaric acid, fumarate, maleic acid, maleate, and the like.

As other monomers for ampholytic polymer 3, from the viewpoint of the solubility of ampholytic polymer 3, N,N'-dimethylacrylamide, butyl (meth)acrylate, and polyethylene glycol mono(meth)acrylate are preferred, and butyl (meth)acrylate is more preferred.

To sufficiently bring out the property of ampholytic polymer 3, the proportion of other repeating units in ampholytic polymer 3 is preferably 50 mol% or less, more preferably 40 mol% or less, relative to the total repeating units of ampholytic polymer 3. The ampholytic polymer 3 does not necessarily contain other repeating units.

When other repeating units (for example, repeating unit formed from butyl (meth)acrylate) of ampholytic polymer 3 are included in ampholytic polymer 3, from the viewpoint of the cryoprotective effect of ampholytic polymer 3, the proportion of other repeating units in ampholytic polymer 3 is preferably 1 mol% or more, more preferably 5 mol% or more, relative to the total repeating units of ampholytic polymer 3.

The ampholytic polymer 3 may be an alternating copolymer, a random copolymer, or a block copolymer. For example, it may be a copolymer having the structure of an alternating copolymer and the structure of a random copolymer. From the viewpoint of easy synthesis, ampholytic polymer 3 is preferably a random copolymer.

### <Number-average molecular weight of ampholytic polymer>

From the viewpoint of the solubility and cryoprotective effect of the ampholytic polymer, the number average molecular weight of the ampholytic polymer is preferably 1,000 to 2,000,000, more preferably 5,000 to 1,000,000. When this number average molecular weight is less than 1,000, the cryoprotective effect of the ampholytic polymer may be weakened, and when this number average molecular weight is greater than 2,000,000, the solubility of the ampholytic polymer in water may decrease. This number average molecular weight is a value calculated from gel permeation chromatography using polyethylene glycol as a standard.

### <Content of ampholytic polymer>

The content of the ampholytic polymer is not particularly limited as long as it has a cryoprotective effect. In order to achieve a good cryoprotective effect, it is preferably 0.1% by mass or more, more preferably 0.5% by mass or more, relative to the entire cryoprotectant of the present invention. In addition, from the viewpoint of the solubility of the ampholytic polymer in water, the aforementioned content is preferably 20% by mass or less, more preferably 10% by mass or less, relative to the entire cryoprotectant of the present invention.

### [Hydrophobic amino acid]

Examples of the hydrophobic amino acid include glycine, alanine, proline, valine, leucine, isoleucine, methionine, phenylalanine, tryptophan, and ectoine. In one embodiment of the present invention, from the viewpoint of cryoprotective effect, the hydrophobic amino acid is preferably at least one selected from the group consisting of proline, ectoine, and valine, more preferably proline or ectoine. In another embodiment of the present invention, from the viewpoint of cryoprotective effect, the hydrophobic amino acid is preferably at least one selected from the group consisting of proline and valine, more preferably proline. The hydrophobic amino acid may be any of D form and L form.

The content of the hydrophobic amino acid is not particularly limited as long as it has a cryoprotective effect. In order to achieve a good cryoprotective effect, it is preferably 0.1% by mass or more, more preferably 0.5% by mass or more, relative to the entire cryoprotectant of the present invention. In addition, from the viewpoint of the solubility of the hydrophobic amino acid in water, the aforementioned content is preferably 10% by mass or less, more preferably 5% by mass or less, relative to the entire cryoprotectant of the present invention.

### [Water-soluble cellulose]

Examples of the water-soluble cellulose include methylcellulose, hydroxyethylcellulose, hydroxypropylcellulose, hydroxypropylmethylcellulose, hydroxyethylmethylcellulose, and carboxymethylcellulose.

From the viewpoint of cryoprotective effect, the water-soluble cellulose is preferably at least one selected from the group consisting of methylcellulose, hydroxyethylcellulose, hydroxypropylcellulose, hydroxypropylmethylcellulose, hydroxyethylmethylcellulose, and carboxymethylcellulose, more preferably at least one selected from the group consisting of methylcellulose, hydroxyethylcellulose, hydroxypropylcellulose, and hydroxypropylmethylcellulose, further preferably methylcellulose.

The viscosity at 20°C of an aqueous solution with a concentration of 2% by mass of the water-soluble cellulose is preferably 0.1 mPa·s or more, more preferably 1 mPa·s or more, further preferably 10 mPa·s or more, and preferably 10,000 mPa· s or less, more preferably 1,000 mPa·s or less, further preferably 100 mPa·s or less, from the viewpoint of ease of handling of the water-soluble cellulose. The aforementioned viscosity is a value measured by a rotational viscometer.

The content of the water-soluble cellulose is not particularly limited as long as it has a cryoprotective effect. In order to achieve a good cryoprotective effect, it is preferably 0.01% by mass or more, more preferably 0.1% by mass or more, relative to the entire cryoprotectant of the present invention. In addition, from the viewpoint of the solubility of the water-soluble cellulose in water, the aforementioned content is preferably 5% by mass or less, more preferably 1% by mass or less, relative to the entire cryoprotectant of the present invention.

### [Water]

The cryoprotectant of the present invention may contain water. As water, purified water, pure water, ion exchange water, and the like are preferred. When the cryoprotectant of the present invention contains water, the content of water is, from the viewpoint of cryoprotective effect, preferably 5% by mass or more, more preferably 10% by mass or more, further preferably 50% by mass or more, particularly preferably 60% by mass or more, and preferably 90% by mass or less, more preferably 80% by mass or less, relative to the entire cryoprotectant of the present invention.

### [Medium]

The cryoprotectant of the present invention may contain a medium. The medium is not particularly limited as long as it is suitable for the cells to be cryopreserved, and general cell culture medium can be used. Examples of the medium include Dulbecco's modified Eagle medium (DMEM), α-MEM medium, Roswell Park Memorial Institute (RPMI) medium, F12 medium, TC199 medium, GMEM medium, and the like. A mixture of these media can be used. In addition, human serum (HSA), bovine serum (e.g., bovine fetal serum (FBS)), glutamine, or antibiotic may be added to the medium as necessary.

### [Other components]

The present invention may contain other components. Examples of other components include reagents generally used in this field to improve viability of cells after freezing and thawing, organic solvent, saccharides, polysaccharides, protein, salt, surfactant, buffer, poly(vinyl alcohol), and the like. Only one kind each of other components may be used, or two or more kinds thereof may be used in combination.

Examples of the organic solvent include dimethyl sulfoxide (DMSO), glycerol, ethylene glycol, propylene glycol, and the like. From the viewpoint of cryoprotective effect, DMSO is preferred.

When the cryoprotectant of the present invention contains DMSO, the content of DMSO is preferably 1% by mass or more, more preferably 5% by mass or more, relative to the entire cryoprotectant of the present invention, from the viewpoint of cryoprotective effect, and preferably 30% by mass or less, more preferably 20% by mass or less, relative to the entire cryoprotectant of the present invention, from the viewpoint of cytotoxicity.

Examples of the saccharides include lactose, sucrose, trehalose, and the like.

Examples of the polysaccharides include alginic acid, pullulan, chitosan, dextran, glucomannan, hydroxyethylstarch, and the like.

Examples of the protein include protein in bovine serum (e.g., bovine serum albumin), protein in bovine fetal serum (FBS), antifreeze peptide, gelatin, casein, protein in human serum (e.g., human serum albumin), growth factor, StemFit (registered trademark, manufactured by Ajinomoto Healthy Supply Co., Inc), and the like.

When the cryoprotectant of the present invention contains FBS, the content of FBS is preferably 5% by mass or more, more preferably 10% by mass or more, further preferably 30% by mass or more, relative to the entire cryoprotectant of the present invention, from the viewpoint of cryoprotective effect, and preferably 80% by mass or less, more preferably 60% by mass or less, further preferably 50% by mass or less, relative to the entire cryoprotectant of the present invention, from the viewpoint of easy handling.

Examples of the salt include salts of inorganic acids such as phosphate, borate, sulfate, Tris hydrochloride, and the like, and salts of organic acids such as acetic acid, citric acid, malic acid, maleic acid, gluconic acid, and the like.

Examples of the surfactant include polyoxyethylene alkyl ether, Tween20, Pluronic (registered trademark), and the like.

As buffers, buffers normally used in this field can be used as long as they do not cause loss of physiological activity such as enzymatic activity and antigenicity of proteins. Examples of the buffer include phosphate buffer, Tris buffer, Good's buffer, glycine buffer, borate buffer, and the like. A mixture of two or more kinds of buffers may be used.

### [Cryopreservation method of cells]

The present invention provides a method for cryopreserving cells (sometimes referred to as "the method of the present invention" in the present specification).

The method of the present invention includes
Step 1 of mixing cells with the cryoprotectant of the present invention, and
Step 2 of freezing the mixture containing the cells and the cryoprotectant.

### <Step 1>

Step 1 is a step of mixing cells with the cryoprotectant of the present invention. For the aforementioned mixing, means generally used in fields that handle cells can be used. The amount of cryoprotectant of the present invention used for 1×10⁴ cells is preferably 1 to 2,000 µL, more preferably 2 to 1,500 µL, further preferably 5 to 1,000 µL, from the viewpoint of cryoprotective effect.

### <Step 2>

Step 2 is a step of freezing the mixture containing cells and the cryoprotectant. The cooling rate for freezing is preferably 0.5 to 10°C/min, more preferably 0.5 to 2°C/min, from the viewpoint of suppressing cell damage. Furthermore, the temperature for cryopreservation after cooling is, for example, -80°C or lower. From the viewpoint of more stable cryopreservation, it is preferable to store the aforementioned mixture frozen in liquid nitrogen or liquid nitrogen vapor.

### [Method for cryopreservation of cells cultured in two dimensions]

A preferred embodiment of the method of the present invention is a method for cryopreserving cells cultured in two dimensions (sometimes referred to as "Method 1 of the present invention" in the present specification).

Method 1 of the present invention includes:
Step A of culturing adherent cells in two dimensions in a medium on a culture substrate;
Step C of mixing the cells cultured in two dimensions with the cryoprotectant of the present invention; and
Step D of freezing a mixture containing the cells cultured in two dimensions on the culture substrate and the cryoprotectant of the present invention. That is, Method 1 of the present invention is, one embodiment of the method of the present invention in which
   the "cells" in the method of the present invention are the "cells cultured in two dimensions",
   the aforementioned Step A is included before the aforementioned Step 1,
   the aforementioned Step 1 is the aforementioned Step C, and
   the aforementioned Step 2 is the aforementioned Step D.

### <Step A>

Step A is a step of two-dimensionally culturing adherent cells in a medium on a culture substrate. As the method of two-dimensional culture, a method generally used in the field of cell culture can be used. There are no particular limitations on the medium, and for example, the above-mentioned ones can be used.

The material of the culture substrate is not particularly limited, and examples thereof include plastic, metal, silicone, glass, and the like. Only one kind of the material of the culture substrate may be used, or two or more kinds thereof may be used in combination. Examples of the material suitable for cell culture include water-resistant and organic-solvent-resistant silicone, glass, and plastic. Examples of the plastic include acrylic polymers such as polymethyl methacrylate and the like, polystyrene, poly(ethylene terephthalate), polycarbonate, and the like. Examples of the silicone include silicone rubbers such polydimethylsiloxane, and the like.

The shape of the culture substrate may be, for example, a container shape, a plate shape, a fiber shape, or a porous shape with holes and grooves formed. Among these, a container shape, a plate shape, and a fiber shape are preferred, and a container shape such as petri dish, flask, or the like is more preferred, for ease of culture of cells cultured in two dimensions.

### <Step B (step of removal of culture supernatant)>

Between Step A and Step C, Step B may be performed to remove the culture supernatant from the cells cultured in two dimensions. In the present specification, "culture supernatant" means "the supernatant liquid obtained when cells are cultured in a medium". The means for removing the culture supernatant are not particularly limited as long as they do not adversely affect the growth of the cells cultured in two dimensions. For example, the culture supernatant can be removed by aspiration using a pipette or aspirator, inversion removal, or centrifugation.

### <Step C>

Step C is a step of mixing the cells cultured in two dimensions with the cryoprotectant of the present invention. For the aforementioned mixing, means generally used in the field of cell culture can be used. The amount of the cryoprotectant of the present invention used for 1×10⁴ cells cultured in two dimensions is preferably 10 to 300 µL, more preferably 20 to 200 µL, further preferably 50 to 200 µL, particularly preferably 50 to 100 µL, from the viewpoint of cryoprotective effect.

### <Step D>

Step D is a step of freezing a mixture containing cells cultured in two dimensions on a culture substrate and the cryoprotectant of the present invention. The cooling rate for freezing is preferably 0.5 to 10°C/min, more preferably 0.5 to 2°C/min, from the viewpoint of suppressing cell damage. The temperature for cryopreservation after cooling is, for example, -80°C or lower. From the viewpoint of more stable cryopreservation, it is preferable to store the aforementioned mixture frozen in liquid nitrogen or liquid nitrogen vapor.

### [Method for cryopreserving cells cultured in three dimensions]

A preferred embodiment of the method of the present invention is a method for cryopreserving cells cultured in three dimensions (sometimes referred to as "Method 2 of the present invention" in the present specification).

Method 2 of the present invention includes
Step E of culturing cells in three dimensions in a medium;
Step G of mixing the cells cultured in three dimensions with the cryoprotectant of the present invention;
Step H of freezing a mixture containing the cells cultured in three dimensions and the cryoprotectant of the present invention. That is, Method 2 of the present invention is one embodiment of the method of the present invention in which

the "cells" in the method of the present invention are the aforementioned "cells cultured in three dimensions",
the aforementioned Step E is included before the aforementioned Step 1,
the aforementioned Step 1 is the aforementioned Step G, and
the aforementioned Step 2 is the aforementioned Step H.

### <Step E>

Step E is a step of culturing cells in three dimensions in a medium. As the method of three-dimensional culture, a method generally used in the field of cell culture can be used. There are no particular limitations on the medium and, for example, the aforementioned one can be used. As a method for producing spheroids, for example, a method using a non-adhesive culture substrate, the hanging drop method, and the like can be mentioned. As a method for producing organoids, for example, an embedding culture method using a scaffold material such as Matrigel and the like can be mentioned.

### <Step F (step of removal of culture supernatant)>

Between Step E and Step G, Step F may be performed in which the cells cultured in three dimensions are transferred to a cryopreservation container and the culture supernatant is removed. The means for removing the culture supernatant are not particularly limited as long as they do not damage the cells cultured in three dimensions. For example, the culture supernatant can be removed by aspiration with a pipette or aspirator, inversion removal, or centrifugation.

### <Step G>

Step G is a step of mixing the cells cultured in three dimensions with the cryoprotectant of the present invention. For the aforementioned mixing, methods generally used in this field can be used. The amount of cryoprotectant of the present invention used for 1×10⁴ cells cultured in three dimensions is preferably 100 to 2,000 µL, more preferably 200 to 1,500 µL, further preferably 500 to 1,000 µL, from the viewpoint of cryoprotective effect.

### <Step H>

Step H is a step of freezing the mixture containing the cells cultured in three dimensions and the cryoprotectant of the present invention. The cooling rate for freezing is preferably 0.5 to 10°C/min, more preferably 0.5 to 2°C/min, from the viewpoint of suppressing cell damage. Furthermore, the temperature for cryopreservation after cooling is, for example, -80°C or lower. From the viewpoint of more stable cryopreservation, it is preferable to store the mixture frozen in liquid nitrogen or liquid nitrogen vapor.

### [Method for cryopreserving cells suspended in medium]

One preferred embodiment of the method of the present invention is a method for cryopreserving cells suspended in a medium (sometimes referred to as "Method 3 of the present invention" in the present specification).

Method 3 of the present invention (i.e., method for cryopreserving cells suspended in medium) includes
Step I of suspending cells in a medium;
Step K of mixing the cells with the cryoprotectant;
Step L of freezing the mixture containing the cells and the cryoprotectant. That is, Method 3 of the present invention is one embodiment of the method of the present invention, in which

"method for cryopreserving cells" in the method of the present invention is the aforementioned "method for cryopreserving cells suspended in a medium",
the aforementioned Step I is included before the aforementioned Step 1,
the aforementioned Step 1 is the aforementioned Step K and
the aforementioned Step 2 is the aforementioned Step L.

### <Step I>

Step I is a step in which the cells are suspended in a medium. The method therefor is not particularly limited, and methods generally used in the field of handling cells can be used. The medium is not particularly limited and, for example, the aforementioned one can be used. The cells may be adherent cells. When adherent cells are used, methods generally used in the field of cell culture can be used to detach the adherent cells from the culture substrate. Examples of such method include, but are not limited to, methods using proteolytic enzymes such as trypsin and the like, and physical detachment methods using cell scrapers and the like. The amount of cells per 1 mL of medium is preferably 1×10⁴ to 1×10⁸ cells, more preferably 1×10⁵ to 1×10⁷ cells.

### <Step J (step of removal of supernatant)>

Between Step I and Step K, Step J may be performed to remove the supernatant from the mixture containing the medium and the cells. The means for removing the supernatant are not particularly limited as long as they do not damage the cells suspended in the medium. For example, the supernatant can be removed by aspiration with a pipette or aspirator, inversion removal, or centrifugation.

### <Step K>

Step K is a step of mixing the cells with the cryoprotectant of the present invention. For the aforementioned mixing, methods generally used in the field handling cells can be used. The amount of cryoprotectant of the present invention used for 1×10⁴ suspended cells is preferably 1 to 20 µL, more preferably 2 to 15 µL, further preferably 5 to 10 µL, from the viewpoint of cryoprotective effect.

### <Step L>

Step L is a step of freezing the mixture containing the cells and the cryoprotectant of the present invention. The cooling rate for freezing is preferably 0.5 to 10°C/min, more preferably 0.5 to 2°C/min, from the viewpoint of suppressing cell damage. Furthermore, the temperature for cryopreservation after cooling is, for example, -80°C or lower. From the viewpoint of more stable cryopreservation, it is preferable to store the mixture frozen in liquid nitrogen or liquid nitrogen vapor.

### [Thawing]

The method for thawing the frozen mixture containing cells (e.g., cells cultured in two dimensions, cells cultured in three dimensions, or cells suspended in a medium) and the cryoprotectant of the present invention is not particularly limited, and methods generally used in this field can be used. To shorten the thawing time, it is preferable to pre-thaw the aforementioned mixture in the range of -80°C to 0°C, add a medium, and then heat the aforementioned mixture.

The medium used in the thawing method preferably contains bovine serum (e.g., fetal bovine serum (FBS)) and/or human serum. Antioxidants and/or apoptosis inhibitors may be added to the medium used in the thawing method or for cell culture after thawing in order to accelerate the recovery of cells from freezing stress. Examples of the antioxidant include, but are not limited to, N-Acetyl-L-cysteine, vitamin A, vitamin C, vitamin E, flavonoid, and derivatives thereof. Examples of the apoptosis inhibitor include, but are not limited to, ROCK inhibitors (e.g., Y27632, AZD-5363, Fasudil, GSK-429286A, H89, Pantoprazole, RKI-1447, Thiazovivin, etc.).

### [Example]

The present invention is specifically described below based on Examples; however, the present invention is not limited to the following Examples.

### <Synthetic Example 1: Synthesis of polymer A>

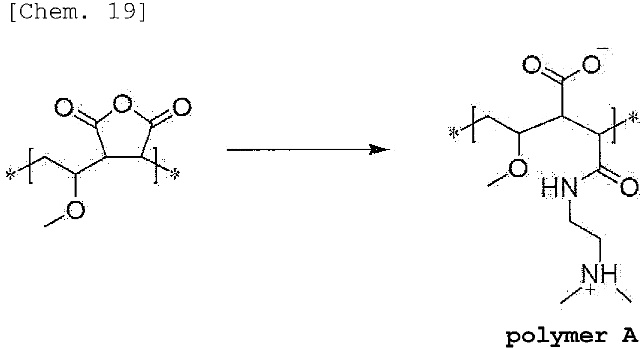

Poly(methyl vinyl ether-alt-maleic anhydride) (hereinafter abbreviated as "pMal", manufactured by Sigma Aldrich, number average molecular weight: 80,000) (2 g) was weighed into a four-necked flask, and tetrahydrofuran (hereinafter abbreviated as "THF") (40 mL) was added. The mixture was stirred at 50°C to dissolve same. Then, 4 equivalents of N,N-dimethylethylenediamine were added relative to the maleic anhydride moiety, and the mixture was stirred at 50°C and reacted. After 1 hr of reaction, the solid was recovered by suction filtration. Thereafter, it was dialyzed against saturated sodium hydrogen carbonate aqueous solution and ion exchanged water (molecular cutoff (hereinafter abbreviated as "MWCO"): 1000), and freeze-dried to obtain polymer A having the aforementioned structure. N,N-dimethylethylenediamine introduction rate: 100% (assigned by ¹H NMR)
¹H NMR of polymer A (DCl+D₂O, 400MHz) δ 3.4-3.5 ppm (-O-CH₃), 2.7-3.1 ppm (-CONH-CH₂-CH₂-), 2.4-2.6 ppm (-N(CH₃)₂), 0.9-1.9 ppm (-CH₂-CH(O-CH₃)-), 1.9-4.0 ppm (polymer main chain)
Yield of polymer A: 2.39 g

The number average molecular weight and polydispersity (weight average molecular weight/number average molecular weight) of the obtained polymer A were determined by gel permeation chromatography using a differential refractometer as the detector. As a pump, PU-2080 manufactured by JASCO Corporation was used, as a detector (differential refractometer ), RI-2031 manufactured by JASCO Corporation was used, and as a detector (UV), UV-2075 was used. As columns, TSKGel G3000PWXL and TSKGel G5000PWXL (column size: 4.6 mm×25 cm), manufactured by Tosoh Corporation, were linked together and used. The developing solvent used was 0.1 M phosphate buffer adjusted to pH 7.4. Measurement conditions were flow rate: 0.6 mL/min, column temperature: 40°C, sample concentration: 2 mg/mL, injection volume: 20 µL. Polyethylene glycol was used as the standard. As a result of gel permeation chromatography, the number average molecular weight of polymer A was 82,600, and the polydispersity thereof was 2.78.

### <Synthetic Example 2: Synthesis of polymer B>

### (1) Synthesis of polymer b

Polymer b, used in the synthesis of polymer B, was synthesized as follows.

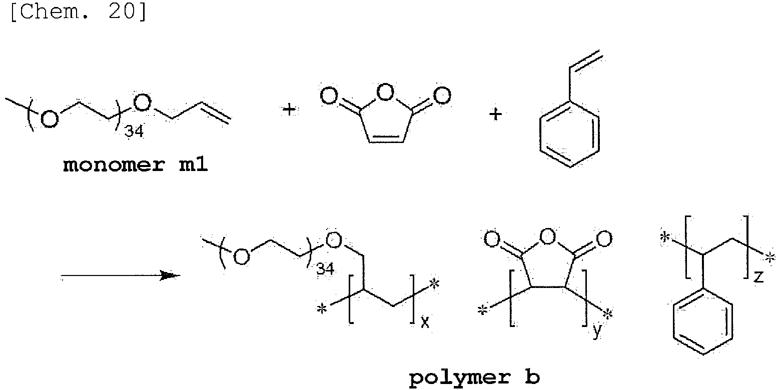

Monomer m1, represented by the aforementioned formula, was synthesized in the same manner as in Synthetic Example 1 described in JP 2002-173351 A. The obtained monomer m1 (1524 g, 1 mol), maleic anhydride (147 g, 1.5 mol), styrene (52 g, 0.5 mol), and toluene (300 g) were weighed, and benzoyl peroxide (4.84 g) was dissolved in toluene (96.8 g) and used as an initiator to copolymerize the aforementioned monomer. After copolymerization, toluene was removed by distillation to obtain polymer b having the aforementioned repeating units.
Molar ratio of repeating units: x/y/z=49.6/49.6/0.8 (assigned by ¹H NMR)
¹H NMR of polymer b (CD₃OD, 400MHz) δ 3.6-3.7 ppm (-O-CH₂-CH₂-O-), 3.4 ppm (-O-CH₃), 1.0-2.3 ppm (-CH₂-CH-), 2.3-3.9 ppm (polymer main chain)

The number average molecular weight of the obtained polymer b was measured by the same method as in Synthetic Example 1. The number average molecular weight of polymer b was 10,000.

### (2) Synthesis of polymer B

Polymer B was synthesized as follows using the obtained polymer b.

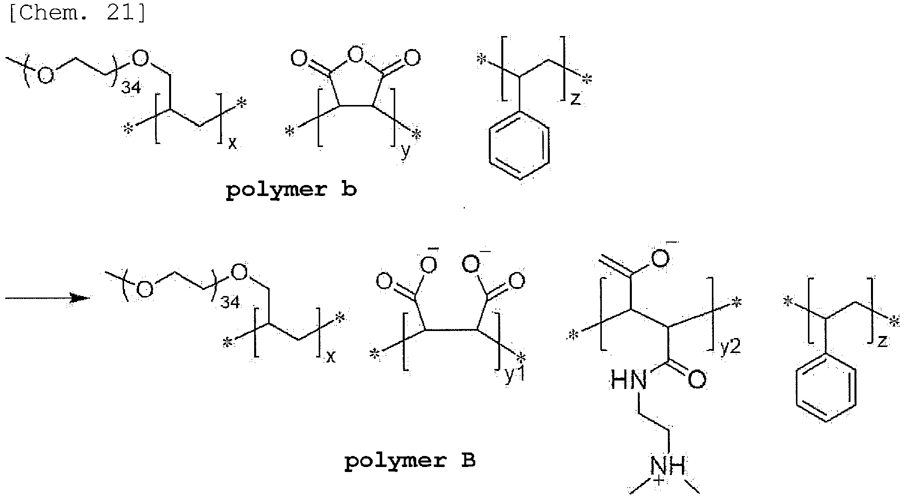

Polymer b (number average molecular weight: 10,000) (2 g) was weighed into a four-necked flask, THF (20 mL) was added, and the mixture was stirred at 50°C to dissolve same. 4 equivalents of N,N-dimethylethylenediamine and triethylamine were added relative to the maleic anhydride unit, and the mixture was stirred at 50°C and reacted. After 4 hr of reaction, Kyoward 600 (100 mg) was added to the reaction mixture, and the obtained mixture was stirred at room temperature (23°C) for 1 hr and filtered by suction. After dialyzing against saturated sodium bicarbonate aqueous solution and ion exchanged water (MWCO: 1000), the mixture was freeze-dried to obtain polymer B having the aforementioned repeating units.
N,N-dimethylethylenediamine introduction rate: 61.6% (assigned by ¹H NMR)
¹H NMR of polymer B (DCl+CD₃OD, 400MHz) δ 3.4-3.9 ppm (-COO-CH₂-CH₂-, -O-CH₂-CH₂-O-), 3.3-3.4 ppm (-O-CH₃), 2.9-3.2 ppm (-N-(CH₃)₂), 1.0-2.4 ppm (-CH₂-CH-), 2.3-3.9 ppm (polymer main chain)
Molar ratio of repeating units: x/y1/y2/z=49.6/19.0/30.6/0.8 (assigned by ¹H NMR)
Yield of polymer B: 1.67 g

The number average molecular weight and polydispersity (weight average molecular weight/number average molecular weight) of the obtained polymer B were measured by the same method as in Synthetic Example 1. The number average molecular weight of polymer B was 17,300, and the polydispersity thereof was 3.34.

### <Synthetic Example 3: Synthesis of polymer C>

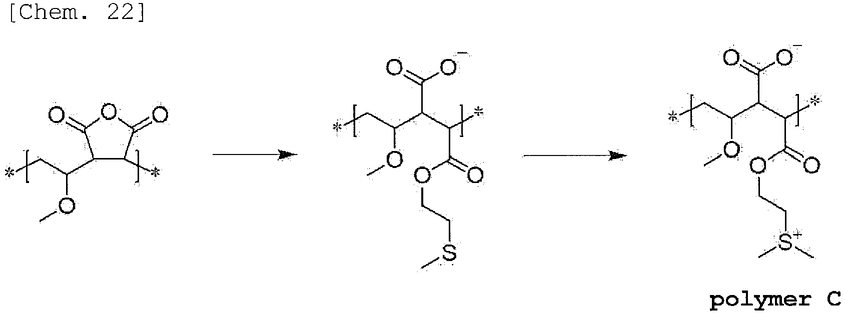

pMal (manufactured by Sigma Aldrich, number average molecular weight: 80,000) (2 g) was weighed into a four-necked flask, THF (40 mL) was added, and the mixture was stirred at 50°C to dissolve same. Then, 2-methylthioethanol and triethylamine were added in amounts of 4 and 4.8 equivalents relative to the maleic anhydride moiety, respectively, and the mixture was stirred and reacted at 50°C. After 1 hr of reaction, the solid was recovered by suction filtration. Thereafter, it was dialyzed against saturated sodium hydrogen carbonate aqueous solution and ion exchanged water (MWCO: 1000), and then freeze-dried.

The obtained dried material (500 mg) was weighed into a four-necked flask, ion exchanged water (4 mL) and ethanol (7 mL) were added to dissolve same at room temperature. After 10 min of nitrogen bubbling (60 mL/min), iodomethane (500 µL, 4.5 equivalents) was added, and the mixture was reacted at room temperature for 19 hr. Thereafter, it was dialyzed against ion exchanged water (MWCO: 1000) and freeze-dried to obtain polymer C having the aforementioned structure.
2-methylthioethanol introduction rate: 100% (assigned by ¹H NMR)
¹H NMR of polymer C (D₂O, 400MHz) δ 4.3-4.6 ppm (-COO-CH₂-), 3.5-3.7 ppm (-CH₂-CH₂-), 3.1-3.3 ppm (-O-CH₃), 2.8-2.9 ppm (-S-(CH₃)₂), 1.3-2.1 ppm (-CH₂-CH-), 2.3-3.9 ppm (polymer main chain)
Yield of polymer C: 0.419 g

The number average molecular weight and polydispersity (weight average molecular weight/number average molecular weight) of the obtained polymer C were measured by the same method as in Synthesis Example 1. The number average molecular weight of polymer C was 83,000, and the polydispersity thereof was 2.80.

### <Synthetic Example 4: Synthesis of polymer D>

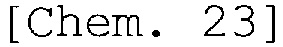

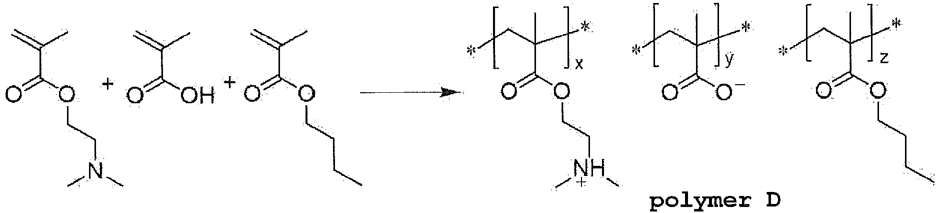

Dimethylaminoethyl methacrylate (665 mg) and methacrylic acid (364 mg) were weighed and dissolved in ion exchanged water (4.2 mL). Also, butyl methacrylate (63 mg), 4-cyano-4-[(dodecylsulfanylthiocarbonyl)sulfanyl]pentanoic acid (7.5 mg), and 4,4'-azobis(4-cyanovaleric acid) (1 mg) were weighed and dissolved in ethanol (2.8 mL). The two solutions were mixed in a four-necked flask, and after 15 min of nitrogen bubbling (20 mL/min), the bubbling was changed to a nitrogen flow (15 mL/min), and the mixture was reacted at 70°C for 20 hr. Thereafter, the mixture was dialyzed against an ion exchanged water/ethanol mixed solvent (volume ratio: 50/50) for 1 day, and ion exchanged water for 2 days (MWCO: 1000), and then freeze-dried to give polymer D having the aforementioned repeating units.
Molar ratio of repeating units: x/y/z=48/45/7 (assigned by ¹H NMR)
¹H NMR of polymer D (D₂O, 400 MHz) δ 4.2-4.5 ppm (2-dimethylaminoethyl methacrylate unit (hereinafter abbreviated as "DMAEMA"): -COO-CH₂-), 4.0-4.2 ppm (butyl methacrylate unit (hereinafter abbreviated as "BMA"): -COO-CH₂-), 3.3-3.7 ppm (-CH₂-CH₂-), 2.7-3.2 ppm (-N-(CH₃)₂), 0.5-2.5 ppm (BMA: -CH₂-CH₂-CH₃, polymer main chain)
Yield of polymer D: 0.700 g

The number average molecular weight and polydispersity (weight average molecular weight/number average molecular weight) of the obtained polymer D were measured by the same method as in Synthesis Example 1. The number average molecular weight of polymer D was 31,600, and the polydispersity thereof was 1.20.

### <Synthetic Example 5: Synthesis of polymer E>

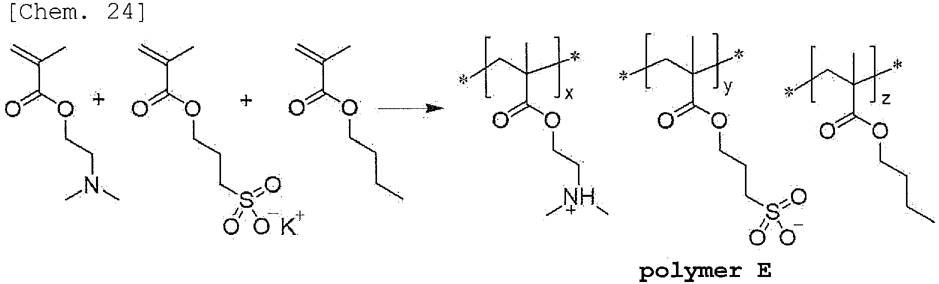

Dimethylaminoethyl methacrylate (411 mg) and 3-sulfopropyl methacrylate potassium salt (643 mg) were weighed and dissolved in ion exchanged water (4.2 mL). Also, butyl methacrylate (39 mg), 4-cyano-4-[(dodecylsulfanylthiocarbonyl)sulfanyl]pentanoic acid (7.5 mg), and 4,4'-azobis(4-cyanovaleric acid) (1 mg) were weighed and dissolved in ethanol (1.8 mL). The two solutions were mixed in a four-necked flask, and after 15 min of nitrogen bubbling (20 mL/min), the bubbling was changed to a nitrogen flow (15 mL/min), and the mixture was reacted at 70°C for 20 hr. Thereafter, the mixture was dialyzed against an ion exchanged water/ethanol mixed solvent (volume ratio: 50/50) for 1 day, and ion exchanged water for 2 days (MWCO: 1000), and then freeze-dried to give polymer E having the aforementioned repeating units.
Molar ratio of repeating units: x/y/z=23/69/8 (assigned by ¹H NMR)
¹H NMR of polymer E (D₂O, 400MHz) δ 4.2-4.5 ppm (DMAEMA: -COO-CH₂-), 4.0-4.2 ppm (3-sulfopropyl methacrylate unit (hereinafter abbreviated as "SPMA"): -COO-CH₂-, BMA: -COO-CH₂-), 3.3-3.7 ppm (DMAEMA: -CH₂-CH₂-), 2.7-3.2 ppm (DMAEMA: -N-(CH₃)₂, SPMA: -CH₂-CH₂-CH₂-), 2.0-2.3 ppm (SPMA: -CH₂-CH₂-CH₂-), 0.5-2.5 ppm (BMA: -CH₂-CH₂-CH₃, polymer main chain)
Yield of polymer E: 0.66 g

The number average molecular weight and polydispersity (weight average molecular weight/number average molecular weight) of the obtained polymer E were measured by the same method as in Synthesis Example 1. The number average molecular weight of polymer E was 11,100, and the polydispersity thereof was 1.37.

### <Synthetic Example 6: Synthesis of polymer F>

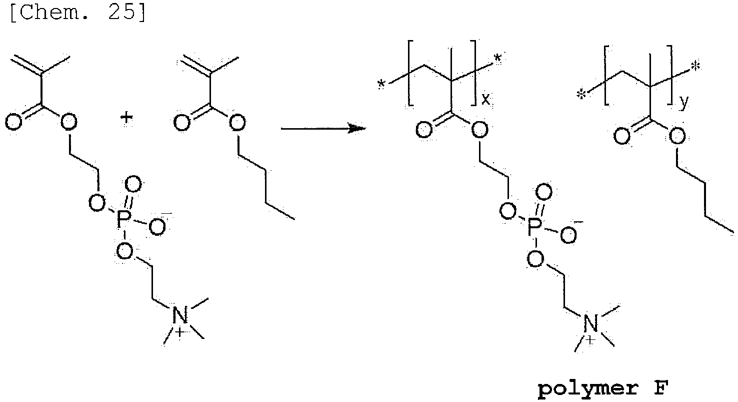

(2-Methacryloyloxy)ethyl phosphorylcholine (975 mg) was weighed and dissolved in ion exchanged water (3.2 mL). Also, butyl methacrylate (117 mg), 4-cyano-4-[(dodecylsulfanylthiocarbonyl)sulfanyl]pentanoic acid (7.5 mg), and 4,4'-azobis(4-cyanovaleric acid) (1 mg) were weighed and dissolved in ethanol (0.8 mL). The two solutions were mixed in a four-necked flask, and after 15 min of nitrogen bubbling (20 mL/min), the bubbling was changed to a nitrogen flow (20 mL/min), and the mixture was reacted at 70°C for 3 hr. Thereafter, the mixture was dialyzed against an ion exchanged water/ethanol mixed solvent (volume ratio: 50/50) for 1 day, and ion exchanged water for 2 days (MWCO: 1000), and then freeze-dried to give polymer F having the aforementioned repeating units.
Molar ratio of repeating units: x/y=88/12 (assigned by ¹H NMR)
¹H NMR of polymer F (D₂O, 400MHz) δ 3.9-4.4 ppm ((2-methacryloyloxy)ethyl phosphorylcholine unit: -COO-CH₂-CH₂-, - PO-CH₂-, BMA: -COO-CH₂-), 3.6-3.8 ppm (-CH₂-CH₂-N-), 1.6-1.8 ppm (BMA: -CH₂-CH₂-CH₃), 1.4-1.6 ppm (BMA: -CH₂-CH₂-CH₃), 1.0-1.2 ppm (BMA: -CH₂-CH₂-CH₃), 0.5-2.5 ppm (polymer main chain)
Yield of polymer F: 0.75 g

The number average molecular weight and polydispersity (weight average molecular weight/number average molecular weight) of the obtained polymer F were measured by the same method as in Synthesis Example 1. The number average molecular weight of polymer F was 23,700, and the polydispersity thereof was 1.25.

### <Synthetic Example 7: Synthesis of polymer G>

### (1) Synthesis of N-(prop-2-enoyl)-L-methionine

N-(prop-2-enoyl)-L-methionine, used in the synthesis of polymer G, was synthesized as follows.

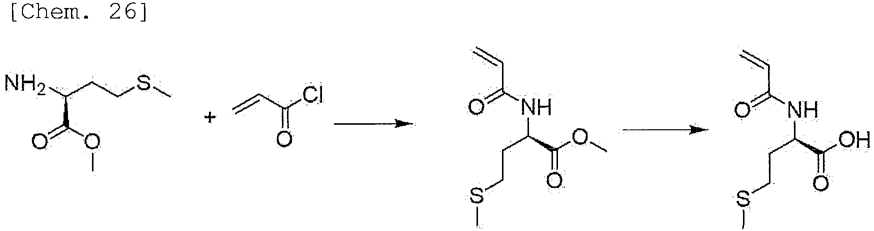

L-methionine methyl ester (10.0 g, 50.0 mmol) was placed in a four-necked flask and dissolved in THF (100 mL). The reaction solution was cooled to 0°C, and triethylamine (15 mL, 108 mmol) and acrylate chloride (5.4 mL, 60 mmol) were added dropwise over 15 min. The reaction solution was then heated to room temperature and stirred for 1 hr. Methanol (2 mL) was added to the reaction solution, and the mixture was stirred at room temperature for another 1 hr. The solvent was evaporated under reduced pressure, methyl tert-butyl ether (hereinafter abbreviated as "MTBE") (200 mL) was added. The organic phase was washed twice with saturated ammonium chloride aqueous solution (50 mL), once with saturated sodium hydrogen carbonate aqueous solution (50 mL), and once with saturated saline solution (100 mL). After drying over magnesium sulfate, the solvent was removed under reduced pressure.

The obtained concentrate was dissolved in acetonitrile (50 mL), and 1N sodium hydroxide aqueous solution (40 mL) was added, and the mixture was stirred at room temperature for 2 hr. The reaction solution was neutralized with 4N hydrochloric acid, and the solvent was removed by vacuum distillation. The pH of the obtained concentrate was adjusted to 3, and then extracted with ethyl acetate (150 mL). The organic phase was washed once with saturated saline solution (100 mL), dried over magnesium sulfate, and the solvent was distilled off under reduced pressure to obtain N-(prop-2-enoyl)-L-methionine.
Yield of N-(prop-2-enoyl)-L-methionine 5.02 g
¹H NMR of N-(prop-2-enoyl)-L-methionine (CD₃OD, 400MHz) δ 6.2-6.4 ppm (CH₂=CH,m,2H), 5.69 ppm (CH₂=CH,t,1H), 4.63 ppm (NH-CH-COOH, t,1H), 2.5-2.6 ppm (CH₂-CH₂-S,m,2H), 2.0-2.2 ppm (CH₂-CH₂-S,m,2H), 2.01 ppm (S-CH₃,s,3H))

### (2) Synthesis of polymer G

Polymer G was synthesized as follows using the obtained N-(prop-2-enoyl)-L-methionine.

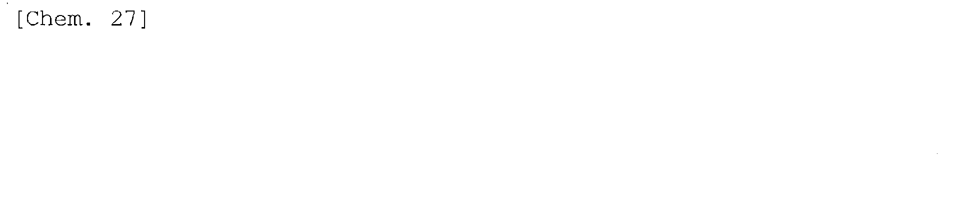

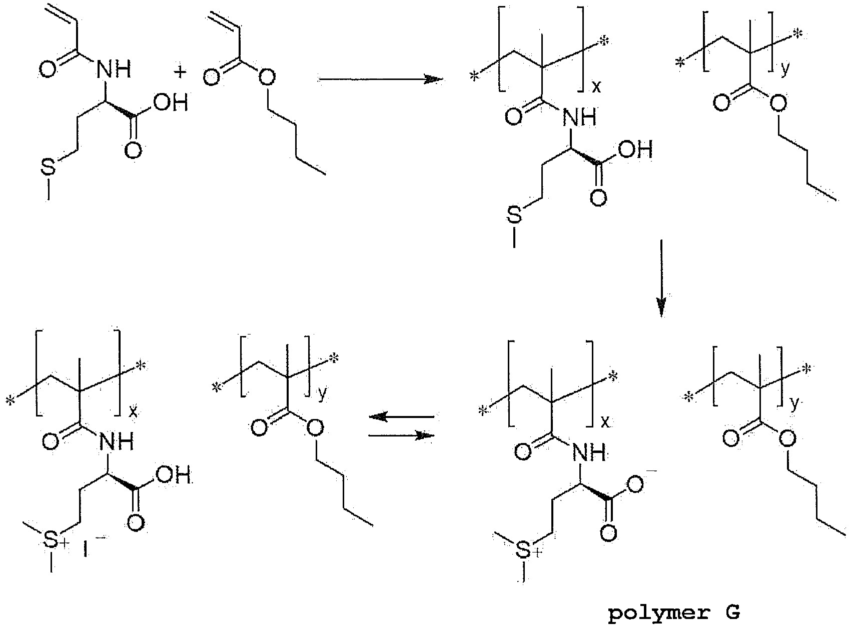

N-(prop-2-enoyl)-L-methionine (944 mg), butyl acrylate (149 mg), 4-cyano-4-[(dodecylsulfanylthiocarbonyl)sulfanyl]pentanoic acid (7.5 mg), and 4,4'-azobis(4-cyanovaleric acid) (1 mg) were weighed and dissolved in N,N-dimethylformamide/ethyl acetate mixed solvent (volume ratio: 4/1) (4.8 mL). The obtained solution was stirred in a four-necked flask, and after 15 min of nitrogen bubbling (20 mL/min), the bubbling was changed to nitrogen flow (20 mL/min), and the mixture was reacted at 70°C for 20 hr. The polymer was then purified by reprecipitation using MTBE/hexane mixed solvent (volume ratio: 50/50) as a poor solvent, and dried under reduced pressure to obtain the precursor of the target product.

The precursor (400 mg) was dissolved in ethanol (4 mL), and nitrogen bubbling (60 mL/min) was performed for 10 min. Iodo methane (400 µL) was added to the obtained mixture, and the mixture was reacted at 23°C for 20 hr. After the reaction, the mixture was dialyzed against an ion exchanged water/ethanol mixed solvent (volume ratio: 50/50) for 1 day and ion exchanged water for 2 days (MWCO: 1000), and then freeze-dried to obtain polymer G having the aforementioned repeating units.
Molar ratio of repeating units: x/y=67/33 (assigned by ¹H NMR) Yield of polymer G: 0.19 g

The number average molecular weight and polydispersity (weight average molecular weight/number average molecular weight) of the obtained polymer G were measured using the same method as in Synthetic Example 1. The number average molecular weight of polymer G was 5,910, and the polydispersity thereof was 1.35.

### <Synthetic Example 8: Synthesis of polymer J>

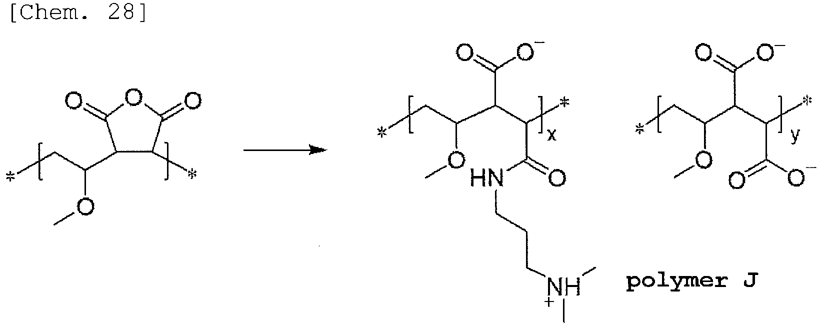

pMal (manufactured by Sigma Aldrich, number average molecular weight: 80,000) (20 g) was weighed into a four-necked flask and dissolved in THF (250 mL). The obtained solution was added dropwise to MTBE (500 mL), and then filtered using filter paper for hirsch funnel No. 5C. After filtration, the solid was recovered by drying under reduced pressure. The above-mentioned procedure was repeated twice to purify pMal.

N,N-dimethyl-1,3-propanediamine (6.0 mL) was added to a four-necked flask, and ion exchanged water (63 mL) was added in an ice bath and mixed to prepare an aqueous solution. Purified pMal (5.0 g) was weighed into a four-necked flask, acetonitrile (hereinafter abbreviated as "ACN") (50 mL) was added to dissolve same to prepare a pMal solution which was then transferred to a dropping funnel. The pMal solution was added dropwise to the aforementioned aqueous solution, and the mixture was reacted for 1 hr. After removing ACN by concentration under reduced pressure, 6M hydrochloric acid was added to the residue to adjust the pH of the mixture to 6.0. After dialyzing against ion exchanged water (MWCO: 3,500), the mixture was freeze-dried to obtain polymer J having the aforementioned structure.
N,N-dimethyl-1,3-propanediamine introduction rate: 76% (assigned by ¹H NMR)
¹H NMR of polymer J (NaOD+D₂O, 400MHz) δ 2.9-3.9 ppm (-O-CH₃, - CONH-CH₂-CH₂-), 2.3-2.9 ppm (-CH₂-CH(O-CH₃)-), 2.1-2.3 ppm (-CONH-CH₂-CH₂-CH₂-), 1.9-2.1 ppm (-N(CH₃)₂), 1.3-1.7 ppm (-CONH-CH₂-CH₂-CH₂-), 1.3-4.0 ppm (polymer main chain)
Molar ratio of repeating units: x/y=76/24 (assigned by ¹H NMR) Yield of polymer J: 5.3 g

The number average molecular weight and polydispersity (weight average molecular weight/number average molecular weight) of the obtained polymer J were determined by gel permeation chromatography using a differential refractometer as the detector. As a pump, PU-2080 manufactured by JASCO Corporation was used, as a detector (differential refractometer), RI-2031 manufactured by JASCO Corporation was used, and as a detector (UV), UV-2075 was used. As columns, TSKGel G3000PWXL and TSKGel G5000PWXL (column size: 4.6 mm × 25 cm), manufactured by Tosoh Corporation, were linked together and used. The developing solvent used was 0.1 M phosphate buffer adjusted to pH 7.4. The measurement conditions were: flow rate: 0.45 mL/min, column temperature: 40°C, sample concentration: 20 mg/mL, injection volume: 100 µL. Polyethylene glycol was used as the standard. As a result of gel permeation chromatography, the number average molecular weight of polymer J was 46,400, and the polydispersity thereof was 2.78.

### <Synthetic Example 9: Synthesis of polymer K>

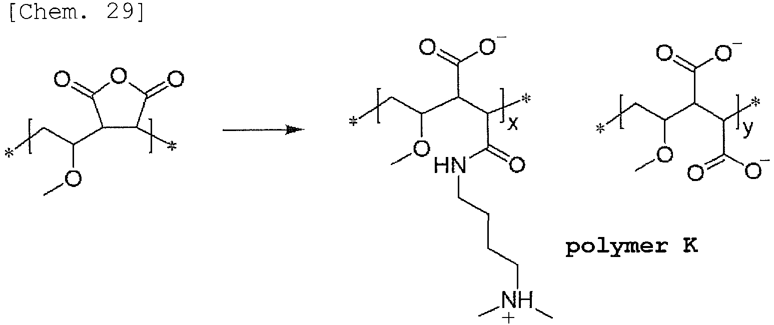

pMal (manufactured by Sigma Aldrich, number average molecular weight: 80,000) (20 g) was weighed into a four-necked flask and dissolved in THF (250 mL). The obtained solution was added dropwise to methyl MTBE (500 mL), and then filtered using filter paper for hirsch funnel No. 5C. After filtration, the solid was recovered by drying under reduced pressure. The above-mentioned procedure was repeated twice to purify pMal.

N,N-dimethyl-1,4-butanediamine (6.2 mL) was added to a four-necked flask, and ion exchanged water (250 mL) was added in an ice bath and mixed to prepare an aqueous solution. Purified pMal (4.5 g) was weighed into a four-necked flask, ACN (45 mL) was added to dissolve same to prepare a pMal solution which was then transferred to a dropping funnel. The pMal solution was added dropwise to the aforementioned aqueous solution, and the mixture was reacted for 1 hr. After removing ACN by concentration under reduced pressure, 6M hydrochloric acid was added to the residue to adjust the pH of the mixture to 6.0. After dialyzing against ion exchanged water (MWCO: 3,500), the mixture was freeze-dried to obtain polymer K having the aforementioned structure.
N,N-dimethyl-1,4-butanediamine introduction rate: 75% (assigned by ¹H NMR)
¹H NMR of polymer K (NaOD+D₂O, 400MHz) δ 2.9-3.9 ppm (-O-CH₃, - CONH-CH₂-CH₂-), 2.3-2.9 ppm (-CH₂-CH(O-CH₃)-), 2.0-2.3 ppm (-CH₂-N(CH₃)₂), 1.8-2.0 ppm (-N(CH₃)₂), 1.0-1.5 ppm (-CONH-CH₂-CH₂-CH₂-CH₂-), 1.3-4.0 ppm (polymer main chain)
Molar ratio of repeating units: x/y=75/25 (assigned by ¹H NMR)
Yield of polymer K: 6.2 g

The number average molecular weight and polydispersity (weight average molecular weight/number average molecular weight) of the obtained polymer K were determined by gel permeation chromatography using a differential refractometer as the detector. As a pump, PU-2080 pump manufactured by JASCO Corporation was used, as a detector (differential refractometer), RI-2031 manufactured by JASCO Corporation was used, and as a detector (UV), UV-2075 was used. As columns, TSKGel G3000PWXL and TSKGel G5000PWXL (column size: 4.6 mm × 25 cm), manufactured by Tosoh Corporation, were linked together and used. The developing solvent used was 0.1 M phosphate buffer adjusted to pH 7.4. The measurement conditions were: flow rate: 0.45 mL/min, column temperature: 40°C, sample concentration: 20 mg/mL, injection volume: 100 µL. Polyethylene glycol was used as the standard. As a result of gel permeation chromatography, the number average molecular weight of polymer K was 46,900, and the polydispersity thereof was 2.72.

### <Example 1: Preparation of cryoprotectant A>

Polymer A (2% by mass), proline (manufactured by Tokyo Chemical Industry Co., Ltd) (2% by mass), methylcellulose (manufactured by Tokyo Chemical Industry Co., Ltd) (0.1% by mass), dimethyl sulfoxide (hereinafter abbreviated as "DMSO") (manufactured by FUJIFILM Wako Pure Chemical Corporation) (10% by mass), bovine fetal serum (hereinafter abbreviated as "FBS") (manufactured by Biosera) (40% by mass), 25mM phosphate buffer (30% by mass), and water (rest) were mixed to prepare cryoprotectant A. As the methylcellulose, a methylcellulose that shows a viscosity of 20 to 30 mPa·s at 20°C when formed into an aqueous solution with its concentration of 2% by mass was used.

### <Examples 2 to 7: Preparation of cryoprotectants B to G>

Cryoprotectants B to G were prepared in the same manner as in Example 1 except that one of polymers B to G was used instead of polymer A.

### <Comparative Example 1: Preparation of cryoprotectant H>

Cryoprotectant H was prepared in the same manner as in Example 1 except that proline and methylcellulose were not used.

### <Comparative Example 2: Preparation of cryoprotectant I>

Cryoprotectant I was prepared in the same manner as in Example 1 except that polymer A and methylcellulose were not used.

### <Examples 8 and 9: Preparation of cryoprotectants J and K>

Cryoprotectants J and K were prepared in the same manner as in Example 1 except that any of polymers J and K was used instead of polymer A.

### <Example 10: Preparation of cryoprotectant L>

Polymer A (4% by mass), proline (manufactured by Tokyo Chemical Industry Co., Ltd) (4% by mass), methylcellulose (manufactured by Tokyo Chemical Industry Co., Ltd) (0.2% by mass), DMSO (manufactured by FUJIFILM Wako Pure Chemical Corporation) (20% by mass), FBS (manufactured by Biosera) (50% by mass), 25mM phosphate buffer (20% by mass), and water (rest) were mixed to prepare cryoprotectant L. As the methylcellulose, a methylcellulose that shows a viscosity of 20 to 30 mPa·s at 20°C when formed into an aqueous solution with its concentration of 2% by mass was used.

The compositions of cryoprotectants A to L obtained in Examples 1 to 10 and Comparative Examples 1 and 2 are shown in Table 1 below.

**[Table 1]**

| | | ampholytic polymer | hydrophobic amino acid | water-soluble cellulose |
|---|---|---|---|---|
| Example 1 | cryoprotectant A | polymer A 2% by mass | proline 2% by mass | methylcellulose 0.1% by mass |
| Example 2 | cryoprotectant B | polymer B 2% by mass | proline 2% by mass | methylcellulose 0.1% by mass |
| Example 3 | cryoprotectant C | polymer C 2% by mass | proline 2% by mass | methylcellulose 0.1% by mass |
| Example 4 | cryoprotectant D | polymer D 2% by mass | proline 2% by mass | methylcellulose 0.1% by mass |
| Example 5 | cryoprotectant E | polymer E 2% by mass | proline 2% by mass | methylcellulose 0.1% by mass |
| Example 6 | cryoprotectant F | polymer F 2% by mass | proline 2% by mass | methylcellulose 0.1% by mass |
| Example 7 | cryoprotectant G | polymer G 2% by mass | proline 2% by mass | methylcellulose 0.1% by mass |
| Comparative Example 1 | cryoprotectant H | polymer A 2% by mass | - | - |
| Comparative Example 2 | cryoprotectant I | - | proline 2% by mass | - |
| Example 8 | cryoprotectant J | polymer J 2% by mass | proline 2% by mass | methylcellulose 0.1% by mass |
| Example 9 | cryoprotectant K | polymer K 2% by mass | proline 2% by mass | methylcellulose 0.1% by mass |
| Example 10 | cryoprotectant L | polymer A 4% by mass | proline 4% by mass | methylcellulose 0.2% by mass |

| | | | | |
|---|---|---|---|---|
| (1) Other components of Examples 1 to 9 and Comparative Examples 1 and 2 DMSO: 10% by mass FBS: 40% by mass 25mM phosphate buffer: 30% by mass rest: water (2) Other components of Example 10 DMSO: 20% by mass FBS: 50% by mass 25mM phosphate buffer: 20% by mass rest: water | | | | |

### <Comparative Example 3: Preparation of cryoprotectant M>

DMSO (10% by mass), FBS (manufactured by Biosera) (10% by mass), and Dulbecco's modified Eagle medium (rest) were mixed to prepare cryoprotectant M.

### <Comparative Example 4: Existing cryoprotectant>

CELLBANKER 1 plus (manufactured by ZENOGEN PHARMA) was used as an existing cryoprotectant.

### <Experimental Example 1: Cryopreservation test of cells cultured in two dimensions>

HepG2 cells suspended in Dulbecco's modified Eagle medium containing 10% by mass of FBS (hereinafter abbreviated as "Medium 1") were seeded by 100 µL into each well of a 96-well plate to 1×10⁴ cells/well, and cultured in two dimensions in a CO₂ incubator at 37°C for 72 hr. Thereafter, Medium 1 was removed from each well, and 50 µL of each cryoprotectant obtained in Examples 1 to 7, or Comparative Example 1 or 2 was added to each well (amount of cryoprotectant used per 1×10⁴ cells to be cryopreserved: about 50 µL).

The cells on the 96-well plate with the added cryoprotectant were cooled at 1°C/min and cryopreserved at - 80°C for 5 days. After cryopreservation, the 96-well plate was left standing at -20°C for 30 min. Then, Medium 1 warmed to 37°C was added to each well by 50 µL, and the plate was left standing at 37°C for 5 min for thawing. After thawing, the cryoprotectant was removed by suction. The cells in each well were washed once with Medium 1, and 100 µL of Medium 1 was added to each well. The cells were cultured for 48 hr, and the obtained cells were subjected to various assays. The results are shown in Table 2.

### (Calculation of viability of cells cultured in two dimensions)

Cell titerglo 2.0 (manufactured by Promega) was added by 100 µL to each well of the 96-well plate after cryopreservation and thawing according to the above-mentioned procedure. After standing at room temperature for 10 min, the emission intensity (hereinafter referred to as "emission intensity after thawing") was measured using a plate reader. The same operation was performed on HepG2 cells before cryopreservation, and the emission intensity (hereinafter referred to as "emission intensity before cryopreservation") was measured. Using the obtained emission intensity after thawing and emission intensity before cryopreservation, the cell viability (%) was calculated using the following formula. 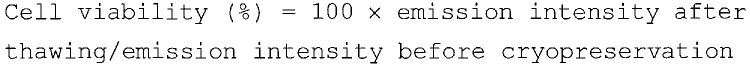 Cell viability (%) = 100 × emission intensity after thawing/emission intensity before cryopreservation

### (Confirmation of morphology (cell detachment and morphological abnormalities) of cells cultured in two dimensions)

The presence or absence of cell detachment and morphological abnormalities of cells in each well of a 96-well plate after cryopreservation and thawing using the above-mentioned procedure were observed using an optical microscope. The morphological abnormalities of cell were evaluated according to the following criteria.

### Evaluation criteria

⊙: no change in cell morphology after thawing compared to before cryopreservation
○: some cells after thawing showed fibroblast-like change in morphology
×: many cells after thawing showed fibroblast-like change in morphology

### (Calculation of urea secretion rate of cells cultured in two dimensions)

After removing Medium 1 from each well of a 96-well plate following the above-mentioned procedure for cryopreservation and thawing, DMEM containing 1 mM ammonium chloride was added to each well. Then, the cells were cultured in a CO₂ incubator at 37°C for 24 hr, the culture supernatant was collected, and the amount of urea in the culture supernatant (hereinafter referred to as "urea amount after thawing") was measured using a Urea Assay Kit (manufactured by Biochain). The same procedure was performed on HepG2 cells before freezing, and the amount of urea in the culture supernatant (hereinafter referred to as "urea amount before cryopreservation") was measured. Using the obtained urea amount after thawing and urea amount before cryopreservation, the urea secretion rate (%) was calculated using the following formula. 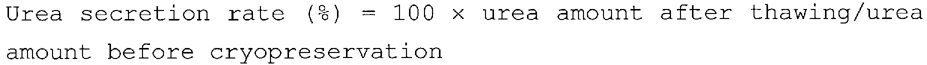 Urea secretion rate (%) = 100 × urea amount after thawing/urea amount before cryopreservation

**[Table 2]**

| | | cell viability (%) | urea secretion rate (%) | cell morphology | |
|---|---|---|---|---|---|
| | | | | cell detached | morphological abnormality |
| Example 1 | cryoprotectant A | 105 | 112 | none | ⊙ |
| Example 2 | cryoprotectant B | 113 | 98 | none | ○ |
| Example 3 | cryoprotectant C | 85 | 141 | none | ○ |
| Example 4 | cryoprotectant D | 115 | 118 | none | ⊙ |
| Example 5 | cryoprotectant E | 108 | 106 | none | ⊙ |
| Example 6 | cryoprotectant F | 102 | 141 | none | O |
| Example 7 | cryoprotectant G | 98 | 121 | none | ○ |
| Comparative Example 1 | cryoprotectant H | 57 | 32 | yes | ○ |
| Comparative Example 2 | cryoprotectant I | 73 | 56 | yes | × |

As shown in Table 2, cryoprotectant A to cryoprotectant G of Examples 1 to 7 maintained higher cell viability for cells cultured in two dimensions compared to cryoprotectant H and cryoprotectant I of Comparative Examples 1 and 2. In addition, using cryoprotectant A to cryoprotectant G, cell detachment and morphological abnormalities in cells cultured in two dimensions can be suppressed, and cell function (urea secretion ability) can be maintained.

### <Experimental Example 2: Cryopreservation test of cells cultured in three dimensions>

HepG2 cells suspended in Medium 1 were seeded by 100 µL into low-adhesion 96-well U-bottom plates (EZ-BindShut (registered trademark) II, manufactured by IWAKI) at 4×10³ cells/well. After centrifugation at 300×g for 5 min, the cells were cultured in three dimensions in a CO₂ incubator at 37°C for 72 hr. After culturing, four obtained spheroids were transferred to one Eppendorf tube, the supernatant was removed until about 100 µL, and the tube was washed with 1000 µL of Medium 1. To the Eppendorf tube after washing, 900 µL of cryoprotectant B obtained in Example 2, cryoprotectant H obtained in Comparative Example 1, cryoprotectant I obtained in Comparative Example 2, or the existing cryoprotectant in Comparative Example 4 was added (amount of cryoprotectant used for 1×10⁴ cells to be cryopreserved: about 560 µL).

The obtained mixture was transferred to a cryotube and placed in BICEL (manufactured by NIHON FREEZER CO., LTD.) and cooled at 1°C/min. The spheroids were cryopreserved at -80°C for 5 days. Thereafter, the removed cryotube was warmed in a 37°C water bath for 2 min to thaw the spheroids. After thawing, the thawed spheroids were added to a 15 mL centrifuge tube containing 9 mL of Medium 1. The supernatant was removed until about 1 mL, and the rest was transferred to an Eppendorf tube. Then, the supernatant was removed until about 100 µL. The spheroids were washed with Medium 1 (1 mL), transferred to a low-adhesion 96-well U-bottom plate, and incubated in a CO₂ incubator at 37°C for 48 hr. The cultured spheroids were then subjected to various assays. The results are shown in Table 3.

### (Calculation of recovery rate of cells cultured in three dimensions)

The number of spheroids placed in cryotube before cryopreservation (hereinafter referred to as "number of spheroids before cryopreservation") and the number of spheroids recovered from cryotubes after cryopreservation and thawing (hereinafter referred to as "number of spheroids after thawing") were measured by optical microscope observation, and the spheroid recovery rate (%) was calculated using the following formula: 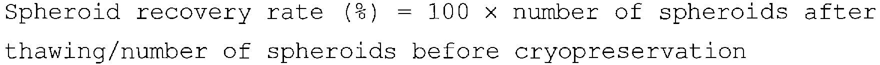 Spheroid recovery rate (%) = 100 × number of spheroids after thawing/number of spheroids before cryopreservation

### (Calculation of viability of cells cultured in three dimensions)

CellTiter-Glo 3D (manufactured by Promega) (50 µL) was added to spheroids cryopreserved, thawed, and cultured for 48 hr according to the above-mentioned procedures (hereinafter referred to as "spheroid 1 after thawing and culture"). The emission intensity (hereinafter referred to as "Emission Intensity 1 after thawing and culture") was measured using Microplate Reader Infinite M1000 PRO (manufactured by TECAN). The same operation was performed on spheroid before cryopreservation, and the emission intensity (hereinafter referred to as "emission intensity 1 before cryopreservation") was measured. Using the obtained emission intensity after thawing and culture, and emission intensity before cryopreservation, the cell viability (%) was calculated using the following formula. 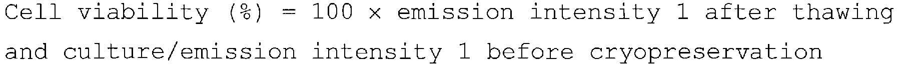 Cell viability (%) = 100 × emission intensity 1 after thawing and culture/emission intensity 1 before cryopreservation

### (Confirmation of morphological changes in cells cultured in three dimensions)

The presence or absence of morphological changes in spheroid 1 after thawing and culture was observed using an optical microscope and evaluated according to the following criteria.

### Evaluation criteria

⊙: no change in the morphology of spheroid 1 after thawing and culture compared to before cryopreservation
O: morphological distortion in part of spheroid 1 after thawing and culture
×: fragmentation of spheroid 1 after thawing and culture

### (Calculation of proportion of dead cells in cells cultured in three dimensions)

Spheroid 1 after thawing and culture was stained with LIVE/DEAD Viability/Cytotoxicity Kit (manufactured by Thermo Fisher SCIENTIFIC), observed with confocal laser scan confocal microscope LSM880 (manufactured by ZEISS), and the proportion of dead cells (%) (= 100 × number of dead cells/total number of cells) was calculated and evaluated according to the following criteria.

### Evaluation criteria

⊙: proportion of dead cells in spheroid 1 after thawing and culturing is less than 20%
O: proportion of dead cells in spheroid 1 after thawing and culturing is 20 to 80%
×: proportion of dead cells in spheroid 1 after thawing and culturing is greater than 80%

**[Table 3]**

| | | spheroid recovery rate (%) | cell viability (%) | morphological change | dead cell |
|---|---|---|---|---|---|
| Example 2 | cryoprotectant B | 100 | 58 | ⊙ | ⊙ |
| Comparative Example 1 | cryoprotectant H | 63 | 53 | ○ | ○ |
| Comparative Example 2 | cryoprotectant I | 63 | 55 | × | ○ |
| Comparative Example 4 | existing cryoprotectant | 58 | 50 | × | ○ |

As shown in Table 3, using cryoprotectant B of Example 2, the spheroid recovery rate and cell viability were improved, and morphological changes and dead cells were suppressed.

### <Experimental Example 3: Cryopreservation test of cells cultured in three dimensions>

HepG2 cells suspended in Medium 1 were seeded by 1000 µL into EZSPHERE (registered trademark) SP microplate 24 well (manufactured by IWAKI) at 1×10⁵ cells/well, and cultured in three dimensions in a CO₂ incubator at 37°C for 72 hr. After culturing, 10 wells of spheroids were transferred to a 15 mL centrifuge tube, the supernatant was removed until about 1000 µL. The tube was washed with 9000 µL of Medium 1, and the culture supernatant was removed again until 1000 µL. Then, 4000 µL of Medium 1 was added to obtain 5000 µL of a spheroid mixture. To the aforementioned spheroid mixture (50 µL) was added 500 µL of cryoprotectant A obtained in Example 1, cryoprotectant M obtained in Comparative Example 3, or the existing cryoprotectant in Comparative Example 4 was added (amount of cryoprotectant used for 1×10⁴ cells to be cryopreserved: about 500 µL).

The obtained mixture was transferred to a cryotube and placed in BICEL (manufactured by NIHON FREEZER CO., LTD.) and cooled at 1°C/min. The spheroids were cryopreserved at -80°C for 5 days. Thereafter, the removed cryotube was warmed in a 37°C water bath for 2 min to thaw the spheroids. After thawing, the thawed spheroids were added to a 15 mL centrifuge tube containing 9 mL of Medium 1. The supernatant was removed until about 1 mL, and the rest was transferred to an Eppendorf tube. Then, the supernatant was removed until about 100 µL. The spheroids were washed with Medium 1 (1 mL), transferred to a low-adhesion 96-well flat bottom plate, and incubated in a CO₂ incubator at 37°C for 24 hr. The cultured spheroids were then subjected to various assays. The results are shown in Table 4.

### (Calculation of viability of cells cultured in three dimensions)

CellTiter-Glo 3D (manufactured by Promega) (50 µL) was added to spheroids cryopreserved, thawed, and cultured for 24 hr according to the above-mentioned procedures (hereinafter referred to as "spheroid 2 after thawing and culture"). The emission intensity (hereinafter referred to as "emission intensity 2 after thawing and culture") was measured using Microplate Reader Infinite M1000 PRO (manufactured by TECAN). The same operation was performed on spheroid before cryopreservation, and the emission intensity (hereinafter referred to as "emission intensity 2 before cryopreservation") was measured. Using the obtained emission intensity after thawing and culture, and emission intensity before cryopreservation, the cell viability (%) was calculated using the following formula. 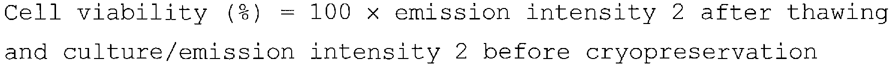 Cell viability (%) = 100 × emission intensity 2 after thawing and culture/emission intensity 2 before cryopreservation

### (Calculation of proportion of dead cells in cells cultured in three dimensions)

Spheroid 2 after thawing and culture was stained with LIVE/DEAD Viability/Cytotoxicity Kit (manufactured by Thermo Fisher SCIENTIFIC), observed with confocal laser scan confocal microscope LSM880 (manufactured by ZEISS), and the proportion of dead cells (%) (= 100 × number of dead cells/total number of cells) was calculated and evaluated according to the following criteria.

### Evaluation criteria

⊙: proportion of dead cells in spheroid 2 after thawing and culturing is less than 20%
O: proportion of dead cells in spheroid 2 after thawing and culturing is 20 to 80%
×: proportion of dead cells in spheroid 2 after thawing and culturing is greater than 80%

**[Table 4]**

| | | cell viability (%) | dead cell |
|---|---|---|---|
| Example 1 | cryoprotectant A | 82 | ⊙ |
| Comparative Example 3 | cryoprotectant M | 61 | × |
| Comparative Example 4 | existing cryoprotectant | 50 | ○ |

As shown in Table 4, using cryoprotectant A of Example 1, the cell viability of the spheroid was improved and dead cells were suppressed.

### <Experimental Example 4: Cryopreservation test of cells suspended in medium>

HepG2 cells, detached from a polystyrene petri dish (manufactured by Corning) after treatment with trypsin-EDTA (manufactured by Sigma Aldrich) at 37°C for 5 min, were suspended in Medium 1 (amount of cells per 1 mL of medium: 1×10⁶ cells). The obtained mixture was centrifuged at 300×g for 5 min, and the supernatant was removed. The HepG2 cells after removal of the supernatant were mixed with cryoprotectant A obtained in Example 1, cryoprotectant M obtained in Comparative Example 3, or the existing cryoprotectant in Comparative Example 4 to prepare a mixture containing HepG2 cells and the cryoprotectant (amount of cryoprotectant used for 1×10⁴ cells to be cryopreserved: about 10 µL, concentration of HepG2 cells in the mixture: 1×10⁶ cells/mL). The obtained mixture was transferred by 1000 µL into cryotubes, then placed in BICEL (manufactured by NIHON FREEZER CO., LTD.), cooled at 1°C/min, and the cells were cryopreserved at -80°C for 5 days. Thereafter, the removed cryotube was warmed in a 37°C water bath for 2 min to thaw the spheroids. The thawed mixture was added to a 15 mL centrifuge tube containing Medium 1 (9 mL), and the mixture was centrifuged at 300×g for 5 min. After suction removal of the medium and cryoprotectant, the obtained cells were suspended in Medium 1 (10 mL). Thereafter, the cell suspension obtained by the aforementioned procedure was subjected to various assays. The results are shown in Table 5.

### (Calculation of cell recovery rate and viability after thawing)

To each cell suspension (100 µL) after cryopreservation and thawing according to the above-mentioned procedure were added 100 µL of Trypan Blue Solution, 0.4% (manufactured by Thermo Fisher SCIENTIFIC), followed by mixing. The number of cells and cell viability of each cell suspension were calculated using Countess 2 automated cell counter (manufactured by Thermo Fisher SCIENTIFIC). Using the obtained number of cells, the cell recovery rate (%) was calculated using the following formula. 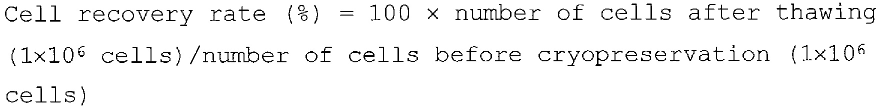 Cell recovery rate (%) = 100 × number of cells after thawing (1×106 cells)/number of cells before cryopreservation (1×106 cells)

**[Table 5]**

| | | cell recovery rate (%) | cell viability (%) |
|---|---|---|---|
| Example 1 | cryoprotectant A | 90 | 97 |
| Comparative Example 3 | cryoprotectant M | 79 | 94 |
| Comparative Example 4 | existing cryoprotectant | 81 | 94 |

As shown in Table 5, using cryoprotectant A of Example 1, the cell recovery rate and cell viability after thawing were improved.

### <Experimental Example 5: Cryopreservation test of cells cultured in two dimensions (w/o step B)>

HepG2 cells suspended in Medium 1 were seeded by 100 µL into each well of a 96-well plate to 1×10⁴ cells/well, and cultured in two dimensions in a CO₂ incubator at 37°C for 72 hr. Thereafter, without removing Medium 1 from each well, 100 µL of cryoprotectant L obtained in Example 10 was added to each well (amount of cryoprotectant used per 1×10⁴ cells to be cryopreserved: about 100 µL).

The cells on the 96-well plate with the added cryoprotectant were cooled at 1°C/min and cryopreserved at - 80°C for 5 days. After cryopreservation, the 96-well plate was left standing at -20°C for 30 min. Then, Medium 1 warmed to 37°C was added to each well by 100 µL, and the plate was left standing at 37°C for 5 min for thawing. After thawing, the cryoprotectant was removed by suction. The cells in each well were washed once with Medium 1, and 100 µL of Medium 1 was added to each well. The cells were cultured for 48 hr, and the obtained cells were subjected to the assays of "Calculation of viability of cells cultured in two dimensions" and "Confirmation of morphology (cell detachment and morphological abnormalities) of cells cultured in two dimensions" in Experimental Example 1. The results are shown in Table 6. In Table 6, the results of Experimental Example 1, which used cryoprotectant A of Example 1 and performed step B (removal of culture supernatant), are compared with the results of Experimental Example 5, which used cryoprotectant L of Example 10 and did not perform step B.

**[Table 6]**

| | | presence or absence of step B | cell viability (%) | cell morphology | |
|---|---|---|---|---|---|
| | | | | cell detached | morphological abnormality |
| Experimental Example 1 | cryoprotectant A of Example 1 | present | 105 | none | ○ |
| Experimental Example 5 | cryoprotectant L of Example 10 | absent | 109 | none | ○ |

As shown in Table 6, the cell viability of Experimental Example 1, which used cryoprotectant A of Example 1 and performed step B (removal of culture supernatant), and the cell viability of Experimental Example 5, which used cryoprotectant L of Example 10 and did not perform step B, were both 100% or higher. Furthermore, in both Experimental Example 1, where step B was performed, and Experimental Example 5, where step B was not performed, cell detachment and morphological abnormalities of the cells cultured in two dimensions could be suppressed.

### <Experimental Example 6: Cryopreservation test of mesenchymal stem cells cultured in two dimensions>

Human subcutaneous adipose-derived mesenchymal stem cells suspended in αMEM medium containing 10% by mass of FBS and 2×10⁻⁷ % by mass of bFGF (hereinafter abbreviated as "Medium 2") were seeded by 100 µL into each well of a 96-well plate to 2.5×10³ cells/well, and cultured in two dimensions in a CO₂ incubator at 37°C for 72 hr. Thereafter, Medium 2 was removed from each well, and 50 µL of each cryoprotectant obtained in Examples 1, 2, 8, or 9, or Comparative Example 3, was added to each well (amount of cryoprotectant used per 1×10⁴ cells to be cryopreserved: about 200 µL).

The cells on the 96-well plate with the added cryoprotectant were cooled at 1°C/min and cryopreserved at - 80°C for 5 days. After cryopreservation, the 96-well plate was left standing at -20°C for 30 min. Then, Medium 2 warmed to 37°C was added to each well by 50 µL, and the plate was left standing at 37°C for 5 min for thawing. After thawing, the cryoprotectant was removed by suction. Then, 100 µL of Medium 2 was added to each well. The cells were cultured for 48 hr, and the obtained cells were subjected to various assays. The results are shown in Table 7.

### (Calculation of viability of mesenchymal stem cells cultured in two dimensions)

Cell titerglo 2.0 (manufactured by Promega) was added by 100 µL to each well of the 96-well plate after cryopreservation and thawing according to the above-mentioned procedure. After standing at room temperature for 10 min, the emission intensity (hereinafter referred to as "emission intensity after thawing") was measured using a plate reader. The same operation was performed on human subcutaneous adipose-derived mesenchymal stem cells before cryopreservation, and the emission intensity (hereinafter referred to as "emission intensity before cryopreservation") was measured. Using the obtained emission intensity after thawing and emission intensity before cryopreservation, the cell viability (%) was calculated using the following formula. 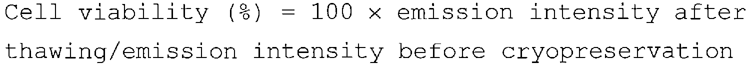 Cell viability (%) = 100 × emission intensity after thawing/emission intensity before cryopreservation

**[Table 7]**

| | | cell viability (%) |
|---|---|---|
| Example 1 | cryoprotectant A | 90 |
| Example 2 | cryoprotectant B | 105 |
| Example 8 | cryoprotectant J | 73 |
| Example 9 | cryoprotectant K | 149 |
| Comparative Example 3 | cryoprotectant M | 22 |

As shown in Table 7, cryoprotectants A, B, J, and K of Examples 1, 2, 8, and 9 all maintained higher cell viability of mesenchymal stem cells cultured in two dimensions, compared to cryoprotectant M of Comparative Example 3.

### [Industrial Applicability]

The cryoprotectant of the present invention is useful for protecting cells (e.g., cells cultured in two dimensions, cells cultured in three dimensions, and cells suspended in medium) during cryopreservation.

This application is based on a patent application No. 2023-174728 filed in Japan, the contents of which are encompassed in full herein.

## Claims

1. A cryoprotectant for cells, comprising an ampholytic polymer, a hydrophobic amino acid, and a water-soluble cellulose, wherein
the ampholytic polymer has a cationic group selected from the group consisting of *-N⁺H₃, *-N⁺(CH₃)H₂, *-N⁺(CH₃)₂H, *-N⁺(CH₃)₃, *-N⁺(CH₃)₂-*, *-S⁺(CH₃)₂, and *-S⁺(CH₃)-* wherein, in the formulas, * is a bonding position, and an anionic group selected from the group consisting of *-CO-O⁻, *-S(O)₂(O⁻), *-OP(O)(OH) (O⁻), and *-O-P(O)(O⁻)-O-* wherein, in the formulas, * is a bonding position.

2. The cryoprotectant according to claim 1, wherein the ampholytic polymer has a repeating structure A represented by the formula (1): 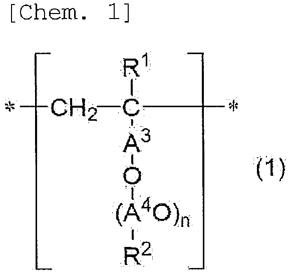
wherein, in the formula (1), * is a bonding position,
R¹ and R² are each independently a hydrogen atom or a methyl group,
A³ is void or a methylene group,
A⁴O is an oxyalkylene group having 2 to 4 carbon atoms, and
n is a number between 0 and 100,
a repeating structure B represented by the formula (2): 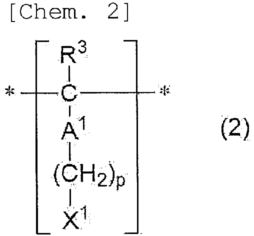
wherein, in the formula (2), * is a bonding position,
R³ is a hydrogen atom or a methyl group,
A¹ is a group selected from the group consisting of *-COO-* and *-CO-NH-* wherein, in the formulas, * is a bonding position,
p is an integer of 1 to 6, and
X¹ is a group selected from the group consisting of *-N⁺H₃, *-N⁺(CH₃)H₂, *-N⁺(CH₃)₂H, *-N⁺(CH₃)₃, and *-S⁺(CH₃)₂ wherein, in the formulas, * is a bonding position, and
a repeating structure C represented by the formula (3): 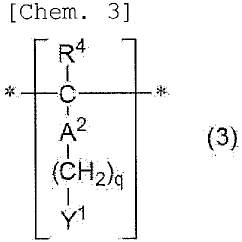
wherein, in the formula (3), * is a bonding position,
R⁴ is a hydrogen atom or a methyl group,
A² is void or a group selected from the group consisting of *-CO-O-* and *-CO-NH-* wherein, in the formulas, * is a bonding position,
q is an integer of 0 to 6, and
Y¹ is a group selected from the group consisting of *-COO⁻, *-S(O)₂(O⁻), *-O-P(O)(OH) (O⁻), and *-C₆H₄-S(O)₂(O⁻) wherein, in the formulas, * is a bonding position and C₆H₄ is a phenylene group, and
a molar ratio of the repeating structure B to the total of the repeating structure B and the repeating structure C is 0.1 or more and 0.9 or less.

3. The cryoprotectant according to claim 2, wherein, in the formula (1), R¹ is a hydrogen atom, R² is a methyl group, A³ is void or a methylene group, A⁴O is an oxyethylene group, and n is a number between 0 and 50,
in the formula (2), R³ is a hydrogen atom, A¹ is a group selected from the group consisting of *-CO-O-* and *-CO-NH-* wherein, in the formulas, * is a bonding position, p is an integer of 2 to 4, and X¹ is a group selected from the group consisting of *-N⁺(CH₃)₂H and *-S⁺(CH₃)₂ wherein, in the formulas, * is a bonding position, and
in the formula (3), R⁴ is a hydrogen atom, A² is void, q is 0, and Y¹ is *-CO-O⁻ wherein, in the formula, * is a bonding position.

4. The cryoprotectant according to claim 3, wherein p is 2.

5. The cryoprotectant according to claim 1, wherein the ampholytic polymer comprises a repeating structure D represented by the formula (4): 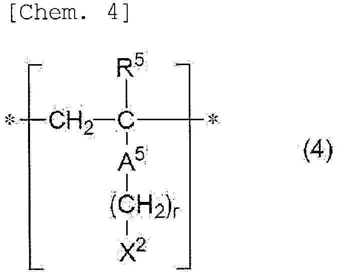
wherein, in the formula (4), * is a bonding position,
R⁵ is a hydrogen atom or a methyl group,
A⁵ is a group selected from the group consisting of *-COO-* and *-CO-NH-* wherein, in the formulas, * is a bonding position,
r is an integer of 1 to 6, and
X² is a group selected from the group consisting of *-N⁺H₃, *-N⁺(CH₃)H₂, *-N⁺(CH₃)₂H, *-N⁺(CH₃)₃, and *-S⁺(CH₃)₂ wherein, in the formulas, * is a bonding position, and
a repeating structure E represented by the formula (5): 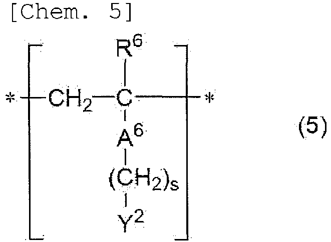
wherein, in the formula (5), * is a bonding position,
R⁶ is a hydrogen atom or a methyl group,
A⁶ is void or a group selected from the group consisting of *-CO-O-*, and *-CO-NH-* wherein, in the formulas, * is a bonding position,
s is an integer of 0 to 6, and
Y² is a group selected from the group consisting of *-COO⁻, *-S (O)₂(O⁻), *-O-P (O)(OH) (O⁻), and *-C₆H₄-S(O)₂(O⁻) wherein, in the formulas, * is a bonding position and C₆H₄ is a phenylene group, and
a molar ratio of the repeating structure D to the total of the repeating structure D and the repeating structure E is 0.1 or more and 0.9 or less.

6. The cryoprotectant according to claim 5, wherein, in the formula (4), R⁵ is a methyl group, A⁵ is *-CO-O-* wherein, in the formula, * is a bonding position, r is 2, and X² is *-N⁺(CH₃)₂H wherein, in the formula, * is a bonding position, and
in the formula (5), R⁶ is a methyl group, A⁶ is void or *-CO-O-* wherein, in the formula, * is a bonding position, s is an integer of 0 to 3, and Y² is *-CO-O⁻ or *-S(O)₂(O⁻) wherein, in the formulas, * is a bonding position.

7. The cryoprotectant according to claim 1, wherein the ampholytic polymer has a group represented by the formula (6): 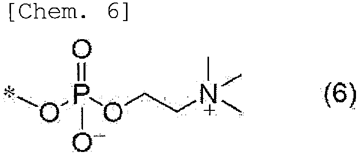 wherein, in the formula (6), * is a bonding position or a group represented by the formula (7): 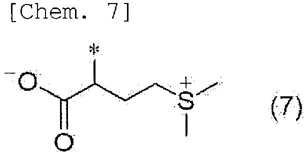 wherein, in the formula (7), * is a bonding position.

8. The cryoprotectant according to any one of claims 1 to 7, wherein the ampholytic polymer has a number average molecular weight of 1,000 to 2,000,000.

9. The cryoprotectant according to any one of claims 1 to 7, wherein the hydrophobic amino acid is proline.

10. The cryoprotectant according to any one of claims 1 to 7, wherein the water-soluble cellulose is at least one selected from the group consisting of methylcellulose, hydroxyethylcellulose, hydroxypropylcellulose, and hydroxypropylmethylcellulose.

11. A method for cryopreserving cells, comprising
Step 1 of mixing cells with the cryoprotectant according to any one of claims 1 to 7, and
Step 2 of freezing the mixture containing the cells and the cryoprotectant.

12. The method according to claim 11, wherein the cells are cells cultured in two dimensions,
the method comprises, before Step 1, a Step A of culturing adherent cells in two dimensions in a medium on a culture substrate,
Step 1 is a Step C of mixing the cells cultured in two dimensions with the cryoprotectant according to any one of claims 1 to 7, and
Step 2 is a Step D of freezing a mixture containing the cells cultured in two dimensions on the culture substrate and the cryoprotectant.

13. The method according to claim 12, comprising, between Step A and Step C, a Step B of removing culture supernatant from the cells cultured in two dimensions.

14. The method according to claim 11, wherein the cells are cells cultured in three dimensions,
the method comprises, before Step 1, a Step E of culturing adherent cells in three dimensions in a medium,
Step 1 is a Step G of mixing the cells cultured in three dimensions with the cryoprotectant according to any one of claims 1 to 7, and
Step 2 is a Step H of freezing a mixture containing the cells cultured in three dimensions and the cryoprotectant.

15. The method according to claim 14, comprising, between Step E and Step G, a Step F of transferring the cells cultured in three dimensions to a cryopreservation container and removing culture supernatant.

16. The method according to claim 11, wherein cells suspended in a medium are cryopreserved,
the method comprises, before Step 1, a Step I of suspending cells in a medium,
Step 1 is a Step K, and
Step 2 is a Step L.

17. The method according to claim 16, comprising, between Step I and Step K, a Step J of removing supernatant from the mixture containing the medium and the cells.
